# EUROPEAN PATENT APPLICATION

(11) **EP 2 009 114 A1**
(43) Date of publication of application: **31.12.2008**
(21) Application number: 07111484.7
(22) Date of filing: 29.06.2007
(51) Int. Cl.: C12Q 1/68, G01N 33/574

(54) **Methods, kits, and compounds for determining responsiveness to treatment of a pathological disorder by epothilones**

(71) Applicant: Bayer Schering Pharma Aktiengesellschaft, 13353 Berlin (DE)
(72) Inventor: The designation of the inventor has not yet been filed

(57) **Abstract**

The invention provides methods, kits and compounds for determining the potential responsiveness of a subject suffering from a pathological disorder, including non-small cell lung cancer (NSCLC), to treatment with an epothilone by analyzing the gene expression profile and/or certain molecular markers in a sample obtained from said subject. The invention further relates to methods, compounds and uses of said compounds for treating subjects suffering from said pathologic disorder, optionally in combination with other therapeutic agents. Also provided are genes and/or proteins encoded by them whose expression level have been determined to differ between epothilone responders and epothilone non-responders.

## Description

### Field of the Invention

The invention provides methods, kits and compounds for determining the potential responsiveness of a subject suffering from a pathological disorder, including non-small cell lung cancer (NSCLC), to treatment with an epothilone by analyzing the gene expression profile and/or certain molecular markers in a sample obtained from said subject. The invention further relates to methods, compounds and uses of said compounds for treating subjects suffering from said pathologic disorder, optionally in combination with other therapeutic agents. Also provided are genes and/or proteins encoded by them whose expression level have been determined to differ between epothilone responders and epothilone non-responders.

### Background of the Invention

Cancer is considered to be a serious and pervasive disease. The National Cancer Institute has estimated that in the United States alone, 1 in 3 people will be afflicted with cancer during their lifetime. Moreover approximately 50% to 60% of people contacting cancer will eventually die from the disease. Lung cancer is one of the most common cancers with an estimated 172,000 new cases projected for 2003 and 157,000 deaths (Jemal et al., 2003, CA Cancer J. Clin., 53, 5-26). Lung carcinomas are typically classified as either small-cell lung carcinomas (SCLC) or non-small cell lung carcinomas (NSCLC). SCLC comprises about 20% of all lung cancers with NSCLC comprising the remaining approximately 80%. NSCLC is further divided into adenocarcinoma (AC) (about 30-35% of all cases), squamous cell carcinoma (SCC) (about 30% of all cases) and large cell carcinoma (LCC) (about 10% of all cases). Additional NSCLC subtypes, not as clearly defined in the literature, include adenosquamous cell carcinoma (ASCC), and bronchioalveolar carcinoma (BAC).

Lung cancer is the leading cause of cancer deaths worldwide, and more specifically non-small cell lung cancer accounts for approximately 80% of all disease cases (Cancer Facts and Figures, 2002, American Cancer Society, Atlanta, p. 11.). There are four major types of non-small cell lung cancer, including adenocarcinoma, squamous cell carcinoma, bronchioalveolar carcinoma, and large cell carcinoma. Adenocarcinoma and squamous cell carcinoma are the most common types of NSCLC based on cellular morphology (Travis et al., 1996, Lung Cancer Principles and Practice, Lippincott-Raven, New York, pps. 361-395). Adenocarcinomas are characterized by a more peripheral location in the lung and often have a mutation in the K-ras oncogene (Gazdar et al., 1994, Anticancer Res. 14:261- 267). Squamous cell carcinomas are typically more centrally located and frequently carry p53 gene mutations (Niklinska et al., 2001, Folia Histochem. Cytobiol. 39:147-148). One particularly prevalent form of cancer, especially among women, is breast cancer. The incidence of breast cancer, a leading cause of death in women, has been gradually increasing in the United States over the last thirty years. In 1997, it was estimated that 181,000 new cases were reported in the U.S., and that 44,000 people would die of breast cancer (Parker et al, 1997, CA Cancer J. CHn. 47:5-27; Chu et al, 1996, J. Nat. Cancer Inst. 88:1571-1579).

Genomic information, in the form of gene expression signatures, has an established capacity to define clinically relevant risk factors in disease prognosis. Recent studies have generated such signatures related to lymph node metastasis and disease recurrence in breast cancer (see West, M. et al., Proc. Natl. Acad. Sci., USA 98, 11462-11467 (2001); Spang, R. et al., In Silico Biol. 2, 0033 (2002); van'T Veer, L. J. et al., Nature 415, 530-536 (2002); van de Vijver, M. J. et al., N. Engl. J. Med. 347, 1999-2009 (2002)), as well as in other cancers (see Pomeroy, S. L. et al., Nature 415, 436-442 (2002); Alizadeh, A. A. et al., Nature 403, 503-511 (2000); Bhattacharjee, A. et al., Proc. Natl. Acad. Sci. USA 98, 13790-13795 (2001); Ramaswamy, S. et al., Proc. Natl. Acad. Sci. 98, 15149-15154 (2001); Golub, T. R. et al., Science 286, 531-537 (1999); Shipp, M. A. et al., Nat. Med. 8, 68-74 (2002); Yeoh, E.-J. et al., Cancer Cell 1, 133-143 (2002)) and non-cancer disease contexts. In spite of considerable research into therapies, these and other cancers remain difficult to diagnose and treat effectively. Accordingly, there is a need in the art for improved methods for classifying and treating such cancers.

In recent decades, a series of highly effective new chemotherapy agents for the therapy of tumors was developed. Despite all of these efforts, the treatment options and the therapeutic window are limited by the high intrinsic toxicity of these pharmaceutical agents.

Only a small portion of the amount of substance administered typically reaches the tumor (Anderson et al., Clin Pharmacokinet 27, 191-201, 1994; Thorpe et al., Breast Canc Res Treat 36, 237-251, 1995), while the maximum amount of substance is taken up by healthy tissue and thus is responsible for many of the undesirable side effects.

For this reason, the selective release of systemically administered chemotherapy agents at the target site always represents a scientific challenge. More recent developments aim at, for example, detoxifying cytostatic agents by conversion into a prodrug form and cleaving the non-toxic prodrug only when reaching the tumor by tumor-associated enzymes. A validation of this concept could be achieved by Bosslet (Bosslet et al., Canc Res 58, 1195-1201, 1998) in the example of a non-toxic prodrug, based on doxorubicin, which was chemically linked to glucuronic acid. In this case, the finding that an elevated lysosomal β-D-glucuronidase activity is observed in the necrotic areas of many tumors was used.

A further development of the understanding relative to the recognition of binding regions, especially in the field of monoclonal antibodies or their fragments from specific tumor antigens, makes it possible to conceive a selective tumor therapy by specific release of an anti-tumor active ingredient at the target site. When the target site is reached, the conjugate binds to the cell surface, and the active ingredient can be released optionally after the entire complex is first internalized.

The successful therapy of solid tumors, especially with monoclonal antibodies, can be limited, however, by an inadequate penetration of the antibody in the tumor as well as the heterogeneous distribution of the corresponding tumor-associated antigen in the tumor tissue.

These limitations thus could be avoided by having the tumor-vascular system be attacked in a specific way. The growth of tumors below a volume of about 2 mm³ is based on a neoangiogenesis. The additional tumor growth is based on an intact vascular system, which ensures the supply with nutrients or disposal of waste products. The selective destruction of this system should therefore result in a necrosis of the tumor. Attacking the vascular system of the tumor offers a number of advantages compared to the direct attack on the tumor itself. In comparison to tumor cells, endothelial cells are easier to access, since no tumor tissue has to be penetrated. The damage of an individual tumor vessel should result in the necrosis of a thousand tumor cells. To damage a tumor vessel, it is not necessary to kill off all endothelial cells. The specific attack of endothelial cells in or near tumors minimizes systemic side effects. Endothelial cells are genetically very stable, such that the probability of a development of resistance against the tumor therapeutic agent is low.

The structural class of the epothilones and analogs thereof offers a possibility of avoiding some of the drawbacks observed in the art. Epothilones represent a novel class of apoptosis inducing anti-tumor compounds. Different publications and reports on study results (e.g. IDrugs, 2002, 5(10):949-954) have proven their anticancer activity. The active strength relative to these cells can be up to 10,000 x greater, compared to chemotherapy agents that are used in clinical practice, such as, for example, taxol, doxorubicin, cis-platinum or camptothecin. Dose regimens are known from the study reports or from other publications, e.g. for epothilones A and B from WO 99/43320.

Epothilone derivatives are known in the art, for example from WO 93/10102, WO 99/02514, WO 2000/066589, WO 2001/027308, and WO 2002/080846.

The epothilones represent a new class of microtubule stabilizing cytotoxic agents (see Gerth, K. et al., J. Antibiot., 1996, 49, 560-3; or Hoefle et al., Angew. Chem. [Applied Chem.], 1996, 108, 1671-1673). These cytotoxic antimitotic agents, block the mitotic spindle of a proliferating cell by binding to the spindle-peptide tubulin, and thus cause apoptosis (K.-H. Altmann, Curr. Opin. Chem. Biol., 2001, 5, 424-431).

The natural products epothilone A and B as well as some of their synthetic derivatives have recently found interest in connection with the treatment of cancer, and a lot of work has been done on their synthesis (K. Nicolaou et al., Angew. Chem., 1998, 110, 2120-2153) and the synthesis of modified structures.

WO 99/07692, WO 99/02514, WO 99/67252, and WO 2004/014919 disclose epothilone derivatives, their synthesis and pharmaceutical use. WO 00/66589 deals with the synthesis and pharmaceutical use of epothilone derivatives having an alkenyl-, alkynyl-, or a cyclic ether containing substituent at the 6(10)-position of the macrocyclic ring. WO 00/49021 discloses epothilone derivatives with a halogen substituent in the 16(3)-position and their synthesis. WO 00/71521 discloses a method for the synthesis of olefinic Epothilones. WO 98/25929 deals with the manufacture of libraries of epothilone analogs.

Nevertheless, with any anticancer drug, including epothilones, one usually observes that certain patients respond well to a given treatment whereas others do not. In this regard, gene profiling has proved a useful tool to differentiate patients that respond to a certain chemotherapeutic treatment, termed responders, from the non-responders. The gene expression profiles of responders versus non-responders for specific chemotherapeutic drugs can predict the response of an individual patient to these drugs and thus allow for a more effective treatment regime. Furthermore, the gene expression profiles with regard to specific drugs can be combined to predict the success of multidrug regimes (see A. Potti et al., Nature Medicine, 2006, 12(11), 1294-1300).

The expression of a large number of genetic markers in different types of cancer cells can be studied via microarray technologies and other methods (see e.g. P.A. Clarke et al., Eur. J. Cancer, 2004, 40, 2560-2591; G. Müller-Hagen et al., Drug Discovery & Development, 2004, 7(3), 291-303). As mentioned above, these studies are useful, since specific genetic markers show differential expression in responders and non-responders to a specific chemotherapeutic agent. However, the response profile of patients to each drug has to be determined individually (see A. Potti et al., Nature Medicine, 2006, 12(11), 1294-1300). Recently, the gene expression patents of patients that are sensitive to EGFR tyrosine inhibitors in the treatment of lung cancer were investigated (J.M. Balko et al., BMC Genomics, 2006, 7, 289), and in another study specific genes that are determinants of sensitivity to paclitaxel were identified (C. Swanton et al., Cancer Cell, 2007, 11, 498-512).

However, as the genetic markers that indicate the sensitivity of patients to a certain chemotherapeutic compound need to be identified for each drug individually, the usefulness of gene expression profiles in determining the individual treatment regime is limited to the drugs for which genetic markers are known.

Thus, although there have been remarkable advances in treating cancer in recent years, there still remains a need in identifying subgroups of patients that respond to a given anticancer drug in order to gain a more rational selection of patients with high probability to benefit from therapy and to avoid side-effects caused by the ineffectiveness of the drug. This is particularly true for the structural class of epothilones.

It is therefore an object of the present invention to provide novel markers that are suitable for predicting the potential responsiveness of a subject to the treatment of a pathological disorder with an epothilone. This and other objects have been achieved by the methods described in the present invention hereinbelow.

### Summary of the Invention

The present inventors have found that the methods, kits and compounds provided herein can be successfully used for identifying subgroups of patients that are expected to respond to a treatment of a pathological disorder such as non-small cell lung cancer with an epothilone.

Hence, in a first aspect the present invention provides a method for determining potential responsiveness of a subject to treatment of a pathologic disorder with an epothilone, wherein said method comprises analyzing in a sample from said subject the expression level of at least one gene or the protein encoded by it, wherein the protein is selected from the group consisting of a cytochrome P (CYP) isoform, microsomal epoxide hydrolase, cytoplasmic epoxide hydrolase, p53, pro-apoptotic Fas-interacting partner (DAXX), neuregulin 1 (NRG1), hepatocyte growth factor (HGF), dystrophin, UGT1A1, UGT1A3, UGT1A4, UGT1A8, UGT1A10, AKR1C2, VEGF, GLUT1, aldolaseA, heme oxygenase, NIP3, PGK1, transferrin, HIF-prolyl hydroxylase, or any combination thereof.

In another aspect, the present invention provides a method for determining potential responsiveness of a subject to treatment of a pathologic disorder with an epothilone, wherein said method comprises analyzing the expression levels of multiple genes in a sample from said subject; and detecting the presence of a pathway deregulation by comparing the expression levels of the genes to a reference profile indicative of pathway deregulation, wherein the presence of pathway deregulation is indicative of a potentially decreased responsiveness of said subject to treatment with said epothilone *(epothilone non-responder).*

Yet another aspect of the present invention relates to a method for determining a marker for potential responsiveness of a subject suffering from a malignant disorder to the treatment of said disorder with an epothilone, wherein the method comprises transferring tumor cells from a subject into nude mice, determining whether the primary tumor, or optionally a passaged xenograft tumor growing on immunodeficient mice or rats, is responsive to the treatment with an epothilone, determining the expression levels of multiple genes or proteins encoded by them in said xenografts or in blood or other tissues from these mice or rats , and identifying a marker protein or a gene coding for said protein by comparing the expression level of said gene or protein encoded by it in at least one xenografts or in blood or other tissues from these mice or rats that is responsive to said epothilone treatment with the expression level of the same gene or protein in at least one xenografts or in blood or other tissues from these mice or rats that is non-responsive to said epothilone treatment, alternatively wherein the expression level is compared to a reference standard in a non-malignant tissue, whereby the marker protein has an altered expression level in the responder compared to the non-responder, or compared to said reference standard expression level.

Another aspect of the invention relates to the use of one or more nucleic acids coding for a marker protein or a fragment thereof in a method for determining potential responsiveness of a subject to treatment of a pathologic disorder with an epothilone, wherein the marker protein is selected from the group consisting of a cytochrome P (CYP) isoform, microsomal epoxide hydrolase, cytoplasmic epoxide hydrolase, p53, pro-apoptotic Fas-interacting partner (DAXX), neuregulin 1 (NRG1), hepatocyte growth factor (HGF), dystrophin, UGT1A1, UGT1A3, UGT1A4, UGT1A8, UGT1A10, AKR1C2, VEGF, GLUT1, aldolaseA, heme oxygenase, NIP3, PGK1, transferrin, HIF-prolyl hydroxylase, or a nucleic acid having a complementary sequence thereto.

Alternatively, the nucleic acid used in the methods of the present invention may hybridize under stringent conditions to the gene coding for said marker protein, or it may have a sequence that is complementary to the coding strand sequence of the gene coding for said marker protein.

A further aspect of the invention relates to the use of an antibody, or any other antibody-derived binding agent against a marker protein as defined above.

In yet another aspect, the present invention relates to kits for determining the potential responsiveness of a subject to treatment of a pathologic disorder with an epothilone, wherein the kits comprise at least one nucleic acid or antibody as described above.

A further aspect of the present invention relates to a method of treating a subject suffering from a pathological disorder, comprising the analysis of the gene expression profile of said subject to determine whether the subject will respond to treatment with an epothilone, and treating the subject with a therapeutically effective amount of an epothilone if the analysis indicates that the subject will respond to the treatment with said epothilone.

In accordance with the present invention, the pathologic disorder is selected from the group consisting of a malignant disorders, tumor diseases, inflammatory diseases, neurodegenerative diseases, angiogenesis-associated diseases, multiple sclerosis, Alzheimer's disease, osteoporosis, bone diseases, or rheumatoid arthritis, and preferably a malignant disorder.

Consequently, the present invention also relates to the use of an epothilone for the manufacture of a pharmaceutical for the treatment of a subject suffering from a pathological disorder selected from malignant disorders, tumor diseases, inflammatory diseases, neurodegenerative diseases, angiogenesis-associated diseases, multiple sclerosis, Alzheimer's disease, osteoporosis, bone diseases, or rheumatoid arthritis, wherein the subject to be treated has been determined to be responsive to a treatment with said epothilone. The pharmaceutical may optionally be administered together with another therapeutic agent that is known to be effective for treating said disease.

Preferably, said malignant disorder is selected from lung cancer, ovarian cancer and breast cancer, whereas most preferably, the malignant disorder is non-small cell lung cancer (NSCLC).

Another aspect of the present invention relates to the use of an epothilone and at least one other therapeutic agent for the manufacture of a pharmaceutical composition for the treatment of a subject suffering from a pathological disorder selected from malignant disorders, tumor diseases, inflammatory diseases, neurodegenerative diseases, angiogenesis-associated diseases, multiple sclerosis, Alzheimer's disease, osteoporosis, bone diseases, or rheumatoid arthritis, wherein the subject to be treated is non-responsive to a treatment with said epothilone. In this case, the at least one other therapeutic agent is capable of modulating the activity level of a pathway that is indicative of a non-responsiveness to the treatment with an epothilone.

In the context of the present invention, all epothilones, epothilone derivatives, and epothilone conjugates known in the art are specifically contemplated herein. For example, epothilones according to the present invention include, but are not limited to, the group consisting of epothilone A, epothilone B, epothilone C, , 13-alkyl-epothilone C derivatives, epothilone D, trans-epothilone D, epothilone E, epothilone F, an effector conjugate of an epothilone, ZK-EPO, ixabepilone (BMS-247550), BMS-310705, EPO-906, patupilone, Kos-862, Kos-1584, Kos-1803, ABJ 879, or a pharmaceutically acceptable salt of these compounds.

### Brief Description of the Figures

**Figure 1****:** Pre-clinical 1° lung cancer study design

**Figure 2a**: Response of non small cell lung cancer (NSCLC) models to treatment with standard chemo therapy

**Figure 2b**: Evaluation of response to 6 chemotherapeutics in non small cell lung cancer (NSCLC) models by conventional preclinical tumor/control (T/C) analysis

**Figure 3**: Evaluation of tumor response

**Figure 4**: Response of 11 NSCLC to Sagopilone

**Figure 5**: Analysis of potential biomarkers

**Figure 6****:** p53 status in sagopilone responder vs non-responder models

**Figure 7**: Epoxide hydrolase expression in Sagopilone responder vs non-responder models

**Figure 8****:** DAXX expression in Sagopilone responder vs non-responder models

**Figure 9**: NRG1 expression in Sagopilone responder vs non-responder models

**Figure 10**: Dystrophin expression in Sagopilone responder vs non-responder models

**Figure 11**: CYP isoform expression in Sagopilone responder vs non-responder models

**Figure 12**: HGF expression in Sagopilone responder vs non-responder models

**Figure 13** Deregulated pathway: UDP-glucuronosyltransferases pathway

**Figure 14**: Deregulated pathway in responder vs non-responder models: Hypoxia / HIF1A pathway

### Detailed Description

The present invention *inter alia* relates to methods, kits and compounds for determining the potential responsiveness of a subject suffering from a pathological disorder to treatment with an epothilone. The potential responsiveness is generally determined by analyzing the expression level of certain marker genes or of the proteins encoded by them in a sample from a subject suffering from said pathological disorder, wherein the altered expression level or mutation status is indicative of a responsiveness or non-responsiveness to such treatment with the epothilone. Accordingly, the present invention allows to identify patient subgroups that are more likely to respond to an epothilone treatment. A further advantage is that the methods of the present invention may help to reduce the incidence and severity of side-effects caused by the administration of the epothilone, and may also help in finding appropriate dosage regimes. Moreover, the markers found to be indicative of a certain responsiveness also offers the possibility to develop a rational treatment regime, including combination therapies with carefully selected therapeutic agents selected in accordance with the determined responsiveness of a patient.

Unless defined otherwise, all technical and scientific terms used herein have the same meaning as commonly understood to one of ordinary skill in the art to which this invention belongs.

The term "or" is used herein to mean, and is used interchangeably with, the term "and/or," unless context clearly indicates otherwise. The term "such as" is used herein to mean, and is used interchangeably, with the phrase "such as but not limited to".

A "patient" or "subject" can mean either a human or non-human animal, preferably a mammal.

The term "expression" is used herein to mean the process by which a polypeptide is produced from DNA. The process involves the transcription of the gene into mRNA and the translation of this mRNA into a polypeptide. Depending on the context in which used, "expression" may refer to the production of RNA, protein or both.

The terms " pathological disorder" and "disease" are used inclusively and refer to any deviation from the normal structure or function of any part, organ or system of the body, or any combination thereof. A specific disease is manifested by characteristic symptoms and signs, including biological, chemical and physical changes, and is often associated with a variety of other factors including, but not limited to, demographic, environmental, employment, genetic and medically historical factors. Certain characteristic signs, symptoms, and related factors can be quantitated through a variety of methods to yield important diagnostic information.

The term "prophylactic" or "therapeutic" treatment refers to administration to the subject of one or more of the subject compositions. If it is administered prior to clinical manifestation of the unwanted condition (e.g., cancer or the metastasis of cancer) then the treatment is prophylactic, i.e., it protects the host against developing the unwanted condition, whereas if administered after manifestation of the unwanted condition, the treatment is therapeutic (i.e., it is intended to diminish, ameliorate or maintain the existing unwanted condition or side effects therefrom).

The term "therapeutic effect" refers to a local or systemic effect in animals, particularly mammals, and more particularly humans caused by a pharmacologically active substance. The term thus means any substance intended for use in the diagnosis, cure, mitigation, treatment or prevention of disease or in the enhancement of desirable physical or mental development and conditions in an animal or human.

The phrase "therapeutically effective amount" means that amount of such a substance that produces some desired local or systemic effect at a reasonable benefit/risk ratio applicable to any treatment. In certain embodiments, a therapeutically-effective amount of a compound will depend on its therapeutic index, solubility, and the like. For example, certain cell lines of the present invention may be administered in a sufficient amount to produce a reasonable benefit/risk ratio applicable to such treatment.

The term "antibody" as used herein is intended to include whole antibodies, e.g., of any isotype (IgG, IgA, IgM, IgE, etc), and includes fragments thereof which are also specifically reactive with a vertebrate, e.g., mammalian, protein. Antibodies can be fragmented using conventional techniques and the fragments screened for utility and/or interaction with a specific epitope of interest. Thus, the term includes segments of proteolytically-cleaved or recombinantly-prepared portions of an antibody molecule that are capable of selectively reacting with a certain protein. Non-limiting examples of such proteolytic and/or recombinant fragments include Fab, F(ab')2, Fab' , Fv, and single chain antibodies (scFv) containing a V[L] and/or V[H] domain joined by a peptide linker. The scFv's may be covalently or non-covalently linked to form antibodies having two or more binding sites. The term antibody also includes polyclonal, monoclonal, or other purified preparations of antibodies and recombinant antibodies, partially or fully humanized antibodies or antibody fragments, as well as antibody-like proteins or oligonucleotides or combinations thereof. Any of the antibody-like proteins and fragments will also be referred to herein as antibody-derived binding moieties.

The term "antineoplastic agent" is used herein to refer to agents that have the functional property of inhibiting a development or progression of a neoplasm or neoplastic cell growth in a human, particularly a malignant (cancerous) lesion, such as a carcinoma, sarcoma, lymphoma, leukemia, or that inhibit/repress tumor endothel and stromal cells, and stem cells.

The terms "overexpressed" or "underexpressed" typically relate to expression of a nucleic acid sequence or protein in a tumor or other tissue at a higher or lower level, respectively, than that level typically observed in a non-tumor cell (i.e., normal control) or, in the context of the present invention, also wherein the expression level differs in a responder relative to a non-responder tumor or other tissue, to a treatment with a given therapeutic agent. In preferred embodiments, the level of expression of a nucleic acid or a protein that is overexpressed in the cancer cell is at least 10%, 20%, 40%, 50%, 60%, 80%, 100%, 150%, 200%, 300%, 400%, 500%, 750%, 1,000%, 2,000%, 5,000%, or 10,000% greater in the cancer cell relative to a normal control.

Alternatively, the term under- and overexpression may also mean that the expression level of said marker protein is increased or decreased by a factor of at least about 1.5, preferably at least about 2, alternatively wherein the expression level of said marker protein is increased by a factor of at least about 3, and preferably at least about 5.

The terms "responder" or "responsiveness" with respect to a therapeutic agent such as an epothilone according to the present invention refer to a sample or a subject wherein the treatment with said therapeutic agent leads to a recovery or at least a stagnation determined in accordance with clinical criteria for the disease. For example, in malignant disorders, a responder will exhibit either a tumor remission/regression (i.e., reduction of tumor volume compared to the begin of treatment) or a stagnation of the disease (essentially no tumor progression, i.e., tumor volume remains essentially constant) after or during treatment with said therapeutic agent. In certain preferred embodiments, tumor remission will refer to a reduction of the tumor volume of at least 1%, 3%, 5%, 10%, 20%, 30%, 40%, 50%, 60%, 70%, 80%, 90%, or 100%.

Similarly, the terms "non-responder" or "non-responsiveness" with respect to a therapeutic agent such as an epothilone according to the present invention refer to a sample or a subject wherein the treatment with said therapeutic agent does not lead to a recovery or at least a stagnation determined in accordance with clinical criteria for the disease. For example, in malignant disorders, a responder will exhibit experience tumor progression (i.e. increase of the tumor volume) after or during treatment with said therapeutic agent.

Tumor regression/remission or tumor growth can, e.g., be determined by a standard tumor volume under the curve (TVUC) test well-known in the art after or during treatment of the sample or subject with a therapeutic agent or a composition of therapeutic agents.

As used herein, the term "pathway" is intended to mean a set of system components involved in two or more sequential molecular interactions that result in the production of a product or activity. A pathway can produce a variety of products or activities that can include, for example, intermolecular interactions, changes in expression of a nucleic acid or polypeptide, the formation or dissociation of a complex between two or more molecules, accumulation or destruction of a metabolic product, activation or deactivation of an enzyme or binding activity. Thus, the term "pathway" includes a variety of pathway types, such as, for example, a biochemical pathway, a gene expression pathway and a regulatory pathway. Similarly, a pathway can include a combination of these exemplary pathway types. A biochemical pathway can include, for example, enzymatic pathways that result in conversion of one compound to another, such as in metabolism, and signal transduction pathways that result in alterations of enzyme activity, polypeptide structure, and polypeptide functional activity. Other pathways may also include a pathway type that is involved in the metabolization or modification of a therapeutic agent in order to prepare the compound for detoxification/elimination from the body.

In some embodiments, the biochemical pathway may be a carbohydrate metabolism pathway, which in a specific embodiment is selected from the group consisting of glycolysis / gluconeogenesis, citrate cycle (TCA cycle), pentose phosphate pathway, pentose and glucuronate interconversions, fructose and mannose metabolism, galactose metabolism, ascorbate and aldarate metabolism, starch and sucrose metabolism, amino sugars metabolism, nucleotide sugars metabolism, pyruvate metabolism, glyoxylate and dicarboxylate metabolism, propionate metabolism, butanoate metabolism, C5-branched dibasic acid metabolism, inositol metabolism and inositol phosphate metabolism. Other pathways include an energy metabolism pathway, which in a specific embodiment is selected from the group consisting of oxidative phosphorylation, ATP synthesis, photosynthesis, carbon fixation, reductive carboxylate cycle (CO2 fixation), methane metabolism, nitrogen metabolism and sulfur metabolism.

In some embodiments, the biochemical pathway is a lipid metabolism pathway, which in a specific embodiment is selected from the group consisting of fatty acid biosynthesis (path 1), fatty acid biosynthesis (path 2), fatty acid metabolism, synthesis and degradation of ketone bodies, biosynthesis of steroids, bile acid biosynthesis, C21 -steroid hormone metabolism, androgen and estrogen metabolism, glycerolipid metabolism, phospholipid degradation, prostaglandin and leukotriene metabolism.

In some embodiments, the biochemical pathway is a nucleotide metabolism pathway, which in a specific embodiment is selected from the group consisting of purine metabolism and pyrimidine metabolism.

In some embodiments, the biochemical pathway is an amino acid metabolism pathway, which in a specific embodiment is selected from the group consisting of glutamate metabolism, alanine and aspartate metabolism, glycine, serine and threonine metabolism, methionine metabolism, cysteine metabolism, valine, leucine and isoleucine degradation, valine, leucine and isoleucine biosynthesis, lysine biosynthesis, lysine degradation, arginine and proline metabolism, histidine metabolism, tyrosine metabolism, phenylalanine metabolism, tryptophan metabolism, phenylalanine, tyrosine and tryptophan biosynthesis, urea cycle, beta- Alanine metabolism, taurine and hypotaurine metabolism, aminophosphonate metabolism, selenoamino acid metabolism, cyanoamino acid metabolism, D-glutamine and D-glutamate metabolism, D-arginine and D-ornithine metabolism, D-alanine metabolism and glutathione metabolism.

In some embodiments, the biochemical pathway is a glycan biosynthesis and metabolism pathway, which in a specific embodiment is selected from the group consisting of N-glycan biosynthesis, N-glycan degradation, O-glycan biosynthesis, chondroitin /heparan sulfate biosynthesis, keratan sulfate biosynthesis, glycosaminoglycan degradation, lipopolysaccharide biosynthesis, clycosylphosphatidylinositol(GPI)-anchor biosynthesis, peptidoglycan biosynthesis, glycosphingolipid metabolism, blood group glycolipid biosynthesis - lactoseries, blood group glycolipid biosynthesis - neo-lactoseries, globoside metabolism and ganglioside biosynthesis.

A gene expression pathway can include, for example, molecules which induce, enhance or repress expression of a particular gene. A gene expression pathway can therefore include polypeptides that function as repressors and transcription factors that bind to specific DNA sequences in a promoter or other regulatory region of the one or more regulated genes. An example of a gene expression pathway is the induction of cell cycle gene expression in response to a growth stimulus.

As used herein, the term "deregulated pathway" is intended to mean a pathway having an altered activity compared to a "normal" regulated pathway. Alterations in activity include, for example, inducing a change in the expression, activity, or physical interactions of a pathway component under a specific condition. The term "deregulated pathway" is used herein to mean a pathway that is either hyperactivated or hypoactivated. A pathway is hyperactivated if it has at least 10%, 20%, 50%, 75%, 100%, 200%, 500%, 1000% greater activity/signaling than the normal pathway. A pathway is hypoactivated if it has at least 10%, 20%, 50%, 75%, 100%, 200%, 500%, 1000% less activity/signaling than the normal pathway. The change in activation status may be due to a mutation of a gene (such as point mutations, deletion, or amplification), changes in transcriptional regulation (such as methylation, phosphorylation, or acetylation changes), or changes in protein regulation (such as translational or post-translational control mechanisms). In certain embodiments, the deregulation is caused by one or more genes or proteins encoded by them that are either upregulated (i.e. overexpressed) or downregulated (i.e., underexpressed) in said deregulated pathway.

In one embodiment, the deregulated pathway is a regulatory pathway. A regulatory pathway can include, for example, a pathway that controls a cellular function under a specific condition. A regulatory pathway controls a cellular function by, for example, altering the activity of a system component or the activity of a biochemical, gene expression or other type of pathway. Specific examples of regulatory pathways include a pathway that activates a cellular function in response to an environmental stimulus of a biochemical system, such as the inhibition of cell differentiation in response to the presence of a cell growth signal and the activation of galactose import and catalysis in response to the presence of galactose and the absence of repressing sugars. The term "component" when used in reference to a network or pathway is intended to mean a molecular constituent of the biochemical system, network or pathway, such as, for example, a polypeptide, nucleic acid, other macromolecule or other biological molecule.

In another embodiment, the deregulated pathway is a signaling pathway. Signaling pathways include MAPK signaling pathways, Wnt signaling pathways, TGF-beta signaling pathways, toll-like receptor signaling pathways, Jak-STAT signaling pathways, second messenger signaling pathways and phosphatidylinositol signaling pathways.

In certain embodiments, the pathway, or the deregulated pathway, contains a tumor suppressor or an oncogene or both. The pathways to which an oncogene or a tumor suppressor gene are assigned are well known in the art, and may be assigned by consulting any of several databases which describe the function of genes and their classification into pathways and/or by consulting the literature (see also Biochemical Pathways: An Atlas of Biochemistry and Molecular Biology. Gerhard Michal (Editor) Wiley, John & Sons, Incorporated, (1998); Biochemistry of Signal Transduction and Regulation, Gerhard Krauss, Wiley, John & Sons, Incorporated, (2003); Signal Transduction. Bastien D. Gomperts, Academic Press, Incorporated (2003)). Databases which may be used include, but are not limited to, http://www.genome.jp/kegg/kegg4.html; Pubmed, OMIM and Entrez at http://www.ncbi.nih.gov; the Swiss-Prot database at http://www.expasy.org/, and many others known in the art.

The term "oncogenic pathway" is used herein to mean a pathway that when hyperactivated or hypoactivated contributes to cancer initiation or progression. In one embodiment, an oncogenic pathway is one that contains an oncogene or a tumor suppressor gene.

Before the present invention and its preferred embodiments are described in further detail, it is to be understood that the invention is not limited to the particular embodiments of the invention described below, as variations of the particular embodiments may be made and still fall within the scope of the appended claims. It is also to be understood that the terminology employed is for the purpose of describing particular embodiments, and is not intended to be limiting in any way.

### Detailed Description of Preferred Embodiments

As mentioned earlier herein, one aspect of the present invention relates to a method for determining potential responsiveness of a subject to treatment of a pathologic disorder with an epothilone, wherein said method comprises analyzing in a sample from said subject the expression level of at least one marker gene (or the protein encoded by it) identified by the present inventors. Potential marker genes for determining the response status of a patient to epothilone treatment are selected from the group consisting of a cytochrome P (CYP) isoform, particularly CYP 2E1, CYP2C9, CYP2C18, and CYP26A1, microsomal epoxide hydrolase, cytoplasmic epoxide hydrolase, p53, pro-apoptotic Fas-interacting partner (DAXX), neuregulin 1 (NRG1), hepatocyte growth factor (HGF), dystrophin, UGT1A1, UGT1A3, UGT1A4, UGT1A8, UGT1A10, AKR1C2, VEGF, GLUT1, aldolaseA, heme oxygenase, NIP3, PGK1, transferrin, and HIF-prolyl hydroxylase.

In the present context, it will be understood that if not explicitly specified, all isoforms of the foregoing marker genes are contemplated herein. The present inventors have found that any of the following observations with regard to the expression level or mutation status of the above-mentioned genes is indicative of a potential responsiveness or decreased responsiveness (i.e. defined herein as non-responsiveness) towards a treatment of a pathological disorder with an epothilone.

For example, it has been observed that all responders to epothilone had a mutation of p53 which lead to a loss of function phenotype for said gene. A p53 loss of function mutation is normally connected to a particularly bad prognosis for malignant disorders. However, it was surprisingly found that tumors having a mutated p53 gene respond particularly well to epothilone treatment (for further details, see Example section). Thus, the presence of a mutated p53 gene leading to a loss of function is indicative of a potential responsiveness of a patient to the treatment with an epothilone.

Other examples for a marker gene indicative of a potential responsiveness of a patient to the treatment with an epothilone is the DAXX gene and the dystrophin gene. The inventors have found that increased gene expression of DAXX and/or dystrophin is indicative of a potential responsiveness of a patient to the treatment with an epothilone

In the context of the methods of the present invention, an increased expression of at least one of the following genes is indicative of a potential decreased responsiveness of a patient to the treatment with an epothilone: a CYP isoform such as CYP 2E1, CYP2C9, CYP2C18, and CYP26A1, microsomal epoxide hydrolase, cytoplasmic epoxide hydrolase, NRG1, and/or HGF. A potentially decreased responsiveness may already be expected if at least one of the aforementioned genes is found to have an increased expression level. Usually, however, it will be observed that more than one, and preferably more than two, three, four or five genes will show an increased expression level in non-responsive patients.

A patient identified to be responsive to a treatment with an epothilone is sometimes referred to herein as an epothilone responder, whereas a patient showing a decreased or no responsiveness as defined herein is sometimes referred to herein as an epothilone non-responder.

Also contemplated herein is a method for determining the potential responsiveness wherein said method comprises analyzing the expression levels of multiple genes in a sample from said subject; and detecting the presence of a pathway deregulation by comparing the expression levels of the genes to a reference profile indicative of pathway deregulation. Alternatively, one may compare the expression profile with a reference profile of a "normal" patient /sample, i.e. unaffected by said pathological disorder. In accordance with the methods of the present invention, the presence of a pathway deregulation is indicative of a potentially decreased responsiveness of said subject to treatment with said epothilone. In other words, such a patient can then be classified as an epothilone non-responder.

Pathways that were found to be deregulated in epothilone non-responder subjects include the UDP glucuronosyl-transferases pathway and the Response to Hypoxia (HIF1A) pathway. Accordingly, methods of the present invention which determine the presence of a deregulated UDP glucuronosyl-transferases pathway and/or a deregulated Response to Hypoxia (HIF1A) pathway are also contemplated herein and are suitable for predicting that the patient is most likely not to respond to a treatment with an epothilone.

In the context of the present invention, a deregulated UDP glucuronosyl-transferases pathway will be present if an increased expression level of at least one of the following genes coding for a protein selected from the group consisting of UGT1A1, UGT1A3, UGT1A4, UGT1A8, UGT1A10, and AKR1C2 is observed. Similarly, a deregulated Response to Hypoxia (HIF1A) pathway will be present when an increased expression level of at least one of the following genes coding for a protein selected from the group consisting of VEGF, GLUT1, aldolaseA, heme oxygenase, NIP3, PGK1, transferrin, and HIF-prolyl hydroxylase is observed.

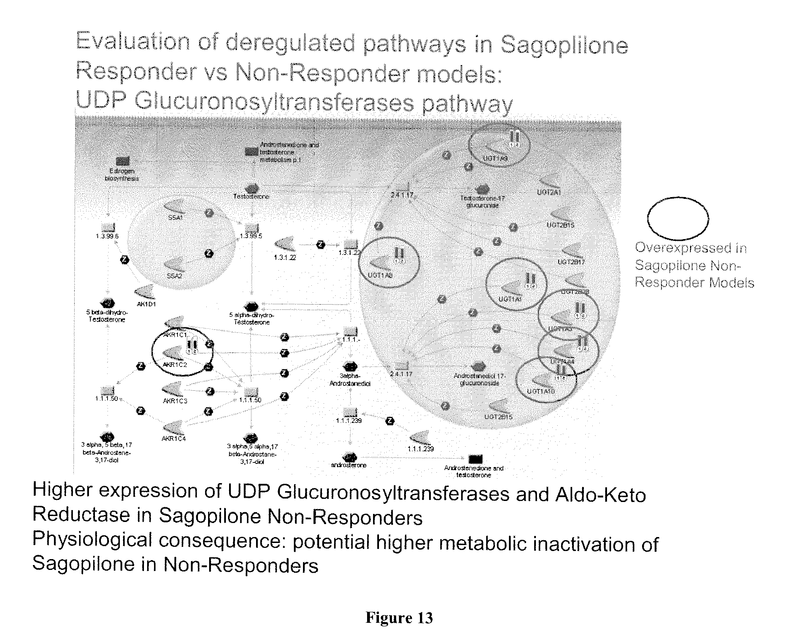

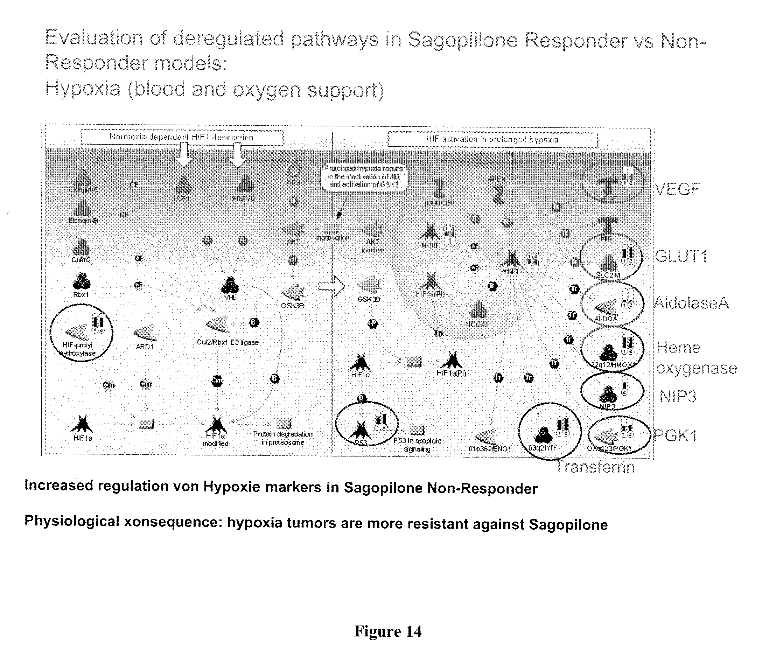

The UDP glucuronosyl-transferases pathway and the Response to Hypoxia (HIF1A) pathways and its major genes involved therein are shown in Figures 13 and 14, respectively.

The determination of the expression level of a gene or a protein encoded by it in accordance with the methods for determining the potential responsiveness to a treatment with an epothilone are preferably carried out *ex vivo,* or even *in vitro.* The determination of expression levels is well-known in the art and some examples are presented in the Example section and the Figures, according to procedures well-known in the art, the expression level is preferably determined from a baseline status, i.e. from a sample which has not (yet) been treated with an epothilone. However, it may also be possible to determine the expression level of one or more than one marker genes in a sample derived from a subject already treated with an epothilone or another therapeutic agent. For best results, however, the expression level should be determined in untreated samples.

A variety of methods for determining the expression level of a one or multiple genes are known in the art and can be used in accordance with the methods of the present invention. One preferred technology for conveniently obtaining a gene expression profile involves the use of a so-called microarray. A number of different microarrays are available in the art, including but not limited to protein-, RNA- or cDNA-based microarrays, SNP arrays, or micro RNA arrays. For example, a gene expression profile can be conveniently determined with the aid of a so-called gene chip, available, e.g., from Affymetrix as described in further detail in the Example section. Gene chip microarrays such as those from Affymetrix consist of small DNA fragments (referred to here as probes), chemically synthesized at specific locations on a coated quartz surface. The precise location where each probe is synthesized is called a feature, and millions of features can be contained on one array. By extracting and labeling nucleic acids from experimental samples, and then hybridizing those prepared samples to the array, the amount of label can be monitored at each feature, enabling a wide range of applications on a whole-genome scale - including gene- and exon-level expression analysis, novel transcript discovery, genotyping, and resequencing.

Alternatively, the expression level may be determined quantitatively by a PCR-based technology, such as quantitative RT-PCR, or by fluorescence in situ hybridization (FISH) techniques (for example, TUNEL for apoptosis).

Of course, the determination of the expression level of a given protein is not limited to nucleic acid based detection methods. Accordingly, other possible ways of determining the expression level of a given protein include the use of western blot technology, ELISA, RIA, immunohistochemistry methods, mass spectrometry technologies such as LC-MS/MS, ESI, or MALDI-ToF.

Yet another alternative way of determining expression levels of marker proteins consists of using antibody-based technologies, which includes protein microarrays and tissue arrays generally known in the art. An antibody as used herein should be understood in its broadest meaning as defined herein, and the person skilled in the art will realize that many antibodies, or binding moieties derived from antibodies may be used in the quantification of the expression level of a given protein. Examples for suitable antibodies include but are not limited to polyclonal, monoclonal, or other purified preparations of antibodies and recombinant antibodies. They also include segments of proteolytically-cleaved or recombinantly-prepared portions of an antibody molecule that are capable of selectively reacting with a certain protein. Non-limiting examples of such proteolytic and/or recombinant fragments include Fab, F(ab')₂, Fab' , Fv, and single chain antibodies (scFv) containing a V[L] and/or V[H] domain joined by a peptide linker. The scFv's may be covalently or non-covalently linked to form antibodies having two or more binding sites.

In the context of the present invention, the antibodies, derivatives or fragments used for the detection of expression levels may furthermore comprise a detectable label allowing their convenient detection and/or quantification. Many suitable labels which can be attached to antibodies and the like are known in the art. For example, preferred detectable labels used in this context are labels containing one or more radioactive atoms, or fluorigenic substrates or fluorescence markers. However, many other suitable detectable labels are known and can equally be employed in the methods of the present invention.

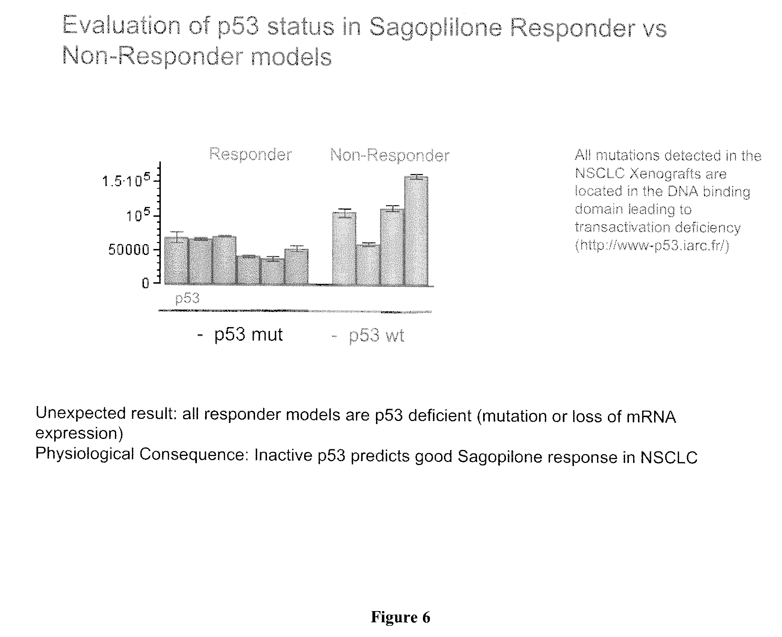

In a further aspect, the present invention also relates to a method for determining or identifying a marker (a gene or the protein encoded by it) for potential responsiveness of a subject suffering from a malignant disorder to the treatment of said disorder with an epothilone. The identification of (novel) marker genes indicative of a potential responsiveness of a subject treated with an epothilone in accordance with this aspect of the invention comprises the step of transferring tumor cells from a subject suffering from a malignant disorder into (immunologically challenged) nude mice, determining whether the primary tumor, or optionally a passaged xenografted tumor growing on mice or rats, is responsive to the treatment with an epothilone, and determining the expression levels of one or, preferably, multiple genes or proteins encoded by them in said a passaged xenografted tumor growing on mice or rats or blood or tissue from the animal . Once the expression data has been acquired, the method further includes the step of identifying a marker protein or a gene coding for said protein by comparing the expression level of said gene or protein encoded by it in at least one a passaged xenografted tumor growing on mice or rats or blood or tissue from the animal that is responsive to said epothilone treatment with the expression level of the same gene or protein in at least one a passaged xenografted tumor growing on mice or rats or blood or tissue from the animal that is non-responsive to said epothilone treatment, wherein the marker protein has an altered expression level in the responder compared to the non-responder. An altered expression level in this context may mean an increased or a decreased expression level. Alternatively, a marker may also have an altered mutation status in a responder sample compared to a non-responder sample. One example for the latter already described herein is the tumor suppressor transcription factor p53 which typically has a different mutation status in responders versus non-responders as shown in Figure 6.

Alternatively, it may be possible to identify a marker by comparing the expression level in a tumor sample obtained from at least one responder a passaged xenografted tumor growing on mice or rats or blood or tissue from the animal with a reference standard obtained from a subject known to be a non-responder to epothilone treatment, non-malignant tissue, or *vice versa,* wherein the marker protein has an altered expression level in the responder compared to the non-responder, or *vice versa.*

The expression levels in accordance with the latter method may also be determined by methods generally known in the art, and may be identical or different to the methods described earlier herein. In particularly preferred embodiments, the expression level of one or multiple genes is determined by the use of a microarray as described herein.

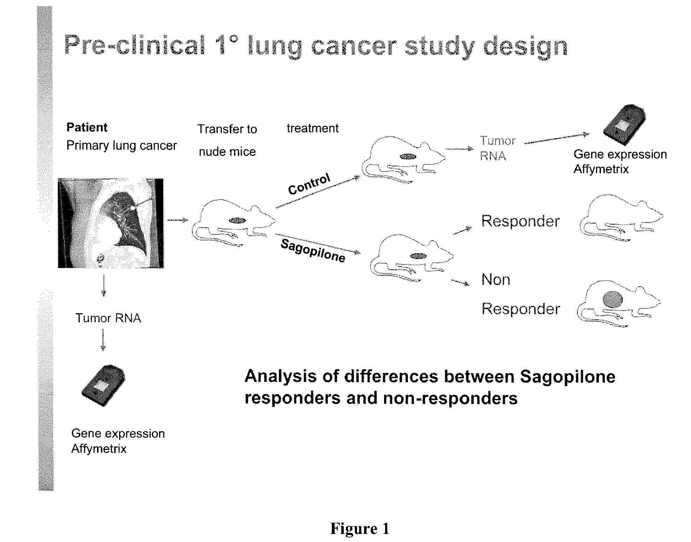

One suitable exemplary setup for the above method for identifying an epothilone response marker is shown in Figure 1, although many modifications of the setup shown in Figure 1 may be apparent to the person skilled in the art. Preferably, the malignant disorder is selected from the group consisting of lung cancer, ovarian cancer or breast cancer. A particularly preferred malignant disorder in the context of the present invention is non-small cell lung cancer (NSCLC).

It will be apparent to those of skill in the art that the diagnostic methods of the present invention will employ nucleic acids or proteins for the detection of an expression level of one or multiple genes or of the proteins encoded by them.

Accordingly, in another aspect, the present invention relates to the use of a nucleic acid coding for a marker protein or a fragment thereof in a method for determining potential responsiveness of a subject to treatment of a pathologic disorder with an epothilone, wherein the marker protein is selected from the group consisting of a cytochrome P (CYP) isoform such as CYP 2E1, CYP2C9, CYP2C18, and CYP26A1, microsomal epoxide hydrolase, cytoplasmic epoxide hydrolase, p53, pro-apoptotic Fas-interacting partner (DAXX), neuregulin 1 (NRG1), hepatocyte growth factor (HGF), dystrophin, UDP glucuronosyl-transferase isoforms UGT1A1, UGT1A3, UGT1A4, UGT1A8, UGT1A10; AKR1C2, VEGF, GLUT1, aldolaseA, heme oxygenase, NIP3, PGK1, transferrin, and HIF-prolyl hydroxylase. Also contemplated herein are of course nucleic acids that have a complementary sequence to said marker protein. The nucleic acids coding for a fragment of said marker proteins must have a sufficient length to specifically identify the gene expression product of interest, and will typically comprise at least 10, preferably at least 15, 20, 25, 30, and in some instances even more bases.

Other nucleic acids that can be used in the method for determining potential responsiveness of a subject to treatment of a pathologic disorder with an epothilone are those that hybridize under stringent conditions to the gene coding for a marker protein as defined hereinabove, or a nucleic acid having a complementary or homologous sequence thereto.

As used herein, the term "complementary" refers to Watson-Crick or Hoogsteen base pairing between nucleotide units of a nucleic acid molecule, and the term "binding" means the physical or chemical interaction between two polypeptides or compounds or associated polypeptides or compounds or combinations thereof.

A "homologous nucleic acid sequence" or "homologous amino acid sequence," or variations thereof, refers to sequences characterized by a homology at the nucleotide level or amino acid level, respectively. Isoforms can be expressed in different tissues of the same organism as a result of, for example, alternative splicing of RNA. Alternatively, isoforms can be encoded by different genes.

In preferred embodiments of this aspect of the invention, the nucleic acid will have more than 60%, more than 70%, more than 80%, more than 90%, more than 93%, 95%, 96%, 97%, 98%, 99% or even 100% homology to the sequence of the (preferably human) gene coding for the marker protein. Most preferably, the nucleic acid sequence will exactly correspond to the sequence of either the coding or the non-coding strand of the gene coding for the marker protein.

As used herein, the phrase "stringent hybridization conditions" refers to conditions under which a probe, primer or oligonucleotide or any other nucleic acid sequence referred to herein will hybridize to its target sequence, but to no other sequences. Stringent conditions are sequence-dependent and will be different in different circumstances. Longer sequences hybridize specifically at higher temperatures than shorter sequences. Generally, stringent conditions are selected to be about 5°C lower than the thermal melting point (Tm) for the specific sequence at a defined ionic strength and pH. The Tm is the temperature (under defined ionic strength, pH and nucleic acid concentration) at which 50% of the probes complementary to the target sequence hybridize to the target sequence at equilibrium. Since the target sequences are generally present at excess, at Tm, 50% of the probes are occupied at equilibrium. Typically, stringent conditions will be those in which the salt concentration is less than about 1.0 M sodium ion, typically about 0.01 to 1.0 M sodium ion (or other salts) at pH 7.0 to 8.3, and the temperature is at least about 30°C for short probes, primers or oligonucleotides (e.g., 10 nt to 50 nt) and at least about 60°C for longer probes, primers and oligonucleotides. Stringent conditions may also be achieved with the addition of destabilizing agents, such as formamide. Stringent conditions are known to those skilled in the art and can be found in Ausubel et al. (eds.), CURRENT PROTOCOLS IN MOLECULAR BIOLOGY, John Wiley & Sons, N.Y. (1989), 6.3.1-6.3.6.

Preferred stringent hybridization conditions in accordance with the nucleic acids of the present invention are hybridization in a high salt buffer comprising 6x SSC, 50 mM Tris-HCl (pH 7.5), 1 mM EDTA, 0.02% PVP, 0.02% Ficoll, 0.02% BSA, and 500 mg/ml denatured salmon sperm DNA at 65 °C, followed by one or more washes in 0.2x SSC, 0.01% BSA at 50°C.

In other preferred embodiments of this aspect, the nucleic acid used for determining epothilone responsiveness will be immobilized on a device such as a RNA- or DNA-based microarray as described herein. Consequently, the nucleic acids employed in these methods may be RNA or DNA, and may be in used as a single strand or a double strand.

While the use of the nucleic acids is not limited to the methods for determining epothilone responsiveness described herein, it is nevertheless preferred that the responsiveness is determined in accordance with the methods of the present invention.

In yet another, but related aspect, the present invention relates to the use of an antibody or an antibody-derived binding moiety in a method for determining potential responsiveness of a subject to treatment of a pathologic disorder with an epothilone. As explained herein, it is possible to detect the expression level on the protein level, i.e., by employing antibodies or antibody-derived binding moieties as defined in further detail hereinabove. The antibodies and antibody-derived binding moieties used in this aspect of the invention must be capable of specifically binding or detecting at least one of the expression products of the marker gene selected from the group of a cytochrome P (CYP) isoform such as CYP 2E1, CYP2C9, CYP2C18, and CYP26A1, microsomal epoxide hydrolase, cytoplasmic epoxide hydrolase, p53, pro-apoptotic Fas-interacting partner (DAXX), neuregulin 1 (NRG1), hepatocyte growth factor (HGF), dystrophin, UDP glucuronosyl-transferase isoforms UGT1A1, UGT1A3, UGT1A4, UGT1A8, UGT1A10; AKR1C2, VEGF, GLUT1, aldolaseA, heme oxygenase, NIP3, PGK1, transferrin, and HIF-prolyl hydroxylase. As already discussed herein, the antibody will preferably be attached to a detectable label such as a radioactive label, or a fluorescent label which allows their convenient detection and quantification.

Similarly to the use of the nucleic acids, the use of the antibodies or antibody-derived binding moieties is also not limited to the methods for determining epothilone responsiveness described herein. However, it is nevertheless preferred that the responsiveness is determined in accordance with the methods of the present invention.

Yet another aspect of the present invention relates to a kit for determining the potential responsiveness of a subject to treatment of a pathologic disorder with an epothilone, wherein the kit comprises at least one nucleic acid as defined herein above. Preferably, however, the kit will comprise more than one nucleic acid, i.e., a multiplicity of different nucleic acids wherein each nucleic acid codes for or hybridizes to a single protein and is therefore suitable for determining the expression level of multiple genes coding for a marker protein. In preferred embodiments, the number of different nucleic acids in the kit will be 2, 3, 4, 5, 6, 7, 8, 9, 10, 11, 12, 13, 14, 15, 16, 17, 18, 19, 20, 21, 22,23, 24, or 25 (i.e., a subset or all proteins/isoforms described herein as a marker for epothilone responsiveness).

Other kits for determining the potential responsiveness of a subject to treatment of a pathologic disorder with an epothilone contemplated herein comprise at least one antibody or antibody-derived binding moiety as defined herein above. Preferably, the kit will comprise more than one antibody or antibody-derived binding moiety. In other words, the kit will comprise a multiplicity of different antibodies or antibody-derived binding moieties wherein each antibody or antibody-derived binding moiety is capable of detecting a defined marker protein. Such a kit is therefore suitable for determining the expression level of multiple marker proteins. In preferred embodiments, the number of different antibodies in the kit will be 2, 3, 4, 5, 6, 7, 8, 9, 10, 11, 12, 13, 14, 15, 16, 17, 18, 19, 20, 21, 22,23, 24, or 25 (i.e., a subset or all proteins/isoforms described herein as a marker for epothilone responsiveness). Of course, the kits may also comprise nucleic acids for certain markers and antibodies or antibody-derived binding moieties in a single kit.

In certain embodiments, the kits of the present invention will comprise the nucleic acids or antibodies or antibody-derived binding moieties immobilized on a microarray as described hereinabove. The kit may optionally comprise further compounds such as buffers, additives, and may additionally comprise instructions for use, for example explaining to the user of the kit how to employ the components of the kit in a method for determining the potential responsiveness of a subject to treatment of a pathologic disorder with an epothilone, or in a method for identifying (further) markers for epothilone responsiveness. The compounds in the kit may be present in a single container or in multiple containers and may be present in solutions (e.g., aqueous or alcoholic), or in dried or lyophilized form, or attached / immobilized on a solid chip, or the like.

The diagnostic methods provided by the present invention allow the identification of subjects suffering from a pathological disorder that are expected to be responsive to a treatment with an epothilone. Similarly, the methods can be used to determine whether a subject is expected to be non-responsive, or at least less responsive to an epothilone treatment.

Based on the valuable information obtained from the diagnostic methods described herein, the present invention therefore provides in a further aspect a method of treating a subject suffering from a pathological disorder, wherein the method comprises the step of analyzing the expression level of at least one gene coding for a marker protein of said subject to determine whether the subject will respond to treatment with an epothilone, and treating the subject with an epothilone if the analysis indicates that the subject will respond to the treatment with said epothilone. Preferably, the method includes the analysis of the gene expression profile of a subject suffering from the pathological disorder, wherein the gene expression profile is determined for a subset or all of the marker genes selected from the group of a cytochrome P (CYP) isoform such as CYP 2E1, CYP2C9, CYP2C18, and CYP26A1, microsomal epoxide hydrolase, cytoplasmic epoxide hydrolase, p53, pro-apoptotic Fas-interacting partner (DAXX), neuregulin 1 (NRG1), hepatocyte growth factor (HGF), dystrophin, UDP glucuronosyl-transferase isoforms UGT1A1, UGT1A3, UGT1A4, UGT1A8, UGT1A10; AKR1C2, VEGF, GLUT1, aldolaseA, heme oxygenase, NIP3, PGK1, transferrin, and HIF-prolyl hydroxylase.

In preferred embodiments of this embodiment of the invention, the analysis is carried out in accordance with the diagnostic methods described herein.

In a different, but related aspect, the present invention also relates to the use of an epothilone for the manufacture of a pharmaceutical for the treatment of a subject suffering from a pathological disorder selected from malignant disorders, tumor diseases, inflammatory diseases, neurodegenerative diseases, angiogenesis-associated diseases, multiple sclerosis, Alzheimer's disease, osteoporosis, bone diseases, or rheumatoid arthritis, wherein the subject to be treated has been determined to be responsive to a treatment with said epothilone. Preferably, but not necessarily, the epothilone responsiveness is determined by any of the diagnostic methods described herein.

Yet another aspect of the present invention relates to the use of an epothilone for the manufacture of a pharmaceutical for the treatment of a tumor characterized by an altered expression of at least one gene selected from the group consisting of a cytochrome P (CYP) isoform, microsomal epoxide hydrolase, cytoplasmic epoxide hydrolase, p53, pro-apoptotic Fas-interacting partner (DAXX), neuregulin 1 (NRG1), hepatocyte growth factor (HGF), dystrophin, UGT1A1, UGT1A3, UGT1A4, UGT1A8, UGT1A10, AKR1C2, VEGF, GLUT1, aldolaseA, heme oxygenase, NIP3, PGK1, transferrin, and HIF-prolyl hydroxylase.

In preferred embodiments, the tumor to be treated with an epothilone is characterized by any one or more than one of the following conditions: p53 loss of function (lower expression and mutation), increased expression of DAXX and/or dystrophin, decreased or at least non-increased expression of a cytochrome P (CYP) isoform, microsomal epoxide hydrolase, cytoplasmic epoxide hydrolase, neuregulin 1 (NRG1), hepatocyte growth factor (HGF), UGT1A1, UGT1A3, UGT1A4, UGT1A8, UGT1A10, AKR1C2, VEGF, GLUT1, aldolaseA, heme oxygenase, NIP3, PGK1, transferrin, or HIF-prolyl hydroxylase compared to the expression level in an epothilone non-responder or in a reference non-tumor tissue.

As shown by the present inventors, tumors characterized according to any one of the aforementioned conditions will at least more likely respond to an epothilone treatment (i.e., tumor remission).

In preferred embodiments of this invention, the tumor disease is selected from the group of lung cancer, ovarian cancer and breast cancer. Most preferably, the tumor disease is a non-small cell lung cancer (NSCLC).

For certain embodiments of the invention, the pharmaceuticals comprising an epothilone according to the present invention may further comprise at least one other therapeutic agent known to be effective in treating the pathological disorder. The at least one other therapeutic agent is preferably known to modulate the activity level of a pathway involved in said disorder. Examples for such other therapeutic agents may preferably be selected from the group of an antineoplastic agent, an anti-inflammatory agent, a neuroprotecting agent, an agent effective in the treatment of angiogenesis-associated diseases, multiple sclerosis, Alzheimer's disease, osteoporosis, bone diseases, or rheumatoid arthritis. Preferably, the at least one other therapeutic agent triggers enhanced cell death (apoptosis) and necrosis.

Suitable examples for an antineoplastic agent include but are not limited to etoposide, cisplatin, carboplatin, gemcitabine, capecitabine, fluorouracil, taxol, docetaxel, navelbine, cetuximab, erlotinib, vinorelbine, and mitomycin, drugs targeting kinases such as EGFR-inhibitors lapatinib, or growth factor antibodies herceptin, cetuximab, VEGFR-kinases such as sorafenib, sutent, IGFR-Kinases, mTOR inhibitors, HDAC-inhibitors such as SAHA, proteasome inhibitors such as velcade, steroid receptor antagonists such as antiestrogens, aromatase inhibitors, antiandrogens, DNA-methyltransferase inhibitors such as azacytidine and the like.

Yet another aspect of the invention relates to the use of an epothilone and at least one other therapeutic agent for the manufacture of a pharmaceutical composition for the treatment of a subject suffering from a pathological disorder selected from malignant disorders, tumor diseases, inflammatory diseases, neurodegenerative diseases, angiogenesis-associated diseases, multiple sclerosis, Alzheimer's disease, osteoporosis, bone diseases, or rheumatoid arthritis, wherein the subject to be treated is known or has been determined to be non-responsive to a treatment with said epothilone in accordance with the diagnostic methods of the present invention. In this aspect, the at least one other therapeutic agent is selected so as to be capable of modulating the activity level of a pathway that is indicative of a non-responsiveness of said subject to the treatment with an epothilone. In other words, the other therapeutic will be capable of affecting a pathway that is responsible for the observed epothilone non-responsiveness. In principle, there are several possible ways how a cell can prevent or at least diminish the effect of an epothilone. One is to upregulate pathways that metabolize the epothilone (i.e. metabolic inactivation of the epothilone), and another possibility is to change the way how a cell reacts to the epothilone, for example, changing the expression level of certain genes leading to lower apoptosis induction. Accordingly, a successful combination therapy is possible by carefully selecting another therapeutic agent that affects and ideally prevents these metabolic adaptations of a cell. In accordance with the findings provided herein, it is thus particularly preferred that the at least one, and preferably at least 2, 3, 4, 5, or 6 other therapeutic agents are chosen from therapeutic agents capable of reducing the expression level of at least one gene coding for a protein selected from the group consisting of a CYP isoform, microsomal epoxide hydrolase, cytoplasmic epoxide hydrolase, NRG1, HGF, UGT1A1, UGTIA3, UGTIA4, UGT1A8, UGT1A10, AKR1C2, VEGF, GLUT1, aldolaseA, heme oxygenase, NIP3, PGK1, transferrin, and HIF-prolyl hydroxylase. Alternatively, the at least one, and preferably at least 2, 3, 4, 5, or 6 other therapeutic agents are chosen from therapeutic agents that are capable of increasing the expression level of a gene coding for DAXX and/or dystrophin.

All pharmaceuticals and pharmaceutical compositions provided herein may optionally comprise further components, such as pharmaceutically acceptable carriers, diluents and/or excipients, and the like.

For any aspect of the present invention, i.e., for all diagnostic as well as therapeutic methods, kits, compositions and uses, the pathologic disorder mentioned herein includes any disorder known to be affected by the administration of an epothilone. Preferred pathologic disorders in the context of the present invention are thus malignant disorders, tumor diseases, inflammatory diseases, neurodegenerative diseases, angiogenesis-associated diseases, multiple sclerosis, Alzheimer's disease, osteoporosis, bone diseases, or rheumatoid arthritis.

Especially preferred is the use of the epothilones of the invention for the treatment of primary tumors and/or metastases that are not operatively accessible, either as monotherapy or in combination with substances that increasingly trigger cell death (apoptosis) and necrosis, so that when cells decompose, it results in an elevated release of normally intracellular, lysosomal enzymes, such as, e.g., glucuronidase, which results in a stronger reaction of the conjugates according to the invention.

With respect to an administration of a combination with the epothilones of the present invention, for example, therapeutic agents are contemplated where the agents are used for the so-called "vascular targeting." These substances result in destruction especially of tumor endothelium, which subsequently results in an elevated necrosis of the tumor because of the deficient supply of nutrients.

Treatment or administration in combination with the above-mentioned substances in this case comprises the simultaneous (both in the mixture and in separate doses) but also the respectively separate administration of the individual components of the combination, for example an alternating administration, as well as administration schemes, in which one component is given as a long-term medication, and the other component is administered in addition at regular or irregular intervals for shorter periods. In this case, the components of the combination can be fed via the same or via different administration paths. In the above-mentioned administrations in combination are preferably those in which the components of the combination achieve an additive action; especially preferred are those administration schemes in which a synergistic action is achieved.

Preferred malignant disorders to be treated with an epothilone include, but are not limited to carcinoma, melanoma, lymphoma, sarcoma, and leukemia. Particularly preferred pathological disorders are selected from lung cancer, ovarian cancer and breast cancer. As has been shown in the Example section, one particularly preferred cancer type that is generally amenable to a treatment with an epothilone is non-small cell lung cancer (NSCLC). In other words, whenever the present invention mentions a pathologic disorder, any of the foregoing disorders or diseases shall be understood to be included.

Likewise, whenever the present invention mentions an epothilone, any compound belonging to the structural class of epothilones, epothilone derivatives, epothilone conjugates and analogs thereof shall be included. For the purposes of the present invention, an epothilone is defined herein as a cyclic molecule with a 16-membered ring and variable substituents and pharmaceutical activity as a cytostatic agent that binds to tubulin (Asnes et al., Anal. Biochem. 1979, 98, 64-73; Job et al., Cellular Pharmacol. 1993, I (Suppl. I), S7-S10; Lichtner et al., PNAS 2001, 98, 11743-11748).

An epothilone in the context of the present invention also includes epothilone conjugates such as those described in WO 2004/050089 and WO 2004/012735, the disclosure of which is incorporated herein in its entirety. Further epothilones are described in WO 93/10102, WO 98/25929,WO 99/02514 , WO 99/07692, WO 99/02514, WO 99/67252, WO 00/49021, WO 00/66589, WO 00/71521, WO 01/027308, WO 02/080846, WO 03/074053, WO 2004/014919, and WO 2005/074901, the disclosure of which is likewise incorporated herein in its entirety. In other words, an "epothilone" as used herein should be understood to include at least the epothilone compounds described in any of the foregoing International applications or its family members.

Thus, the present invention also includes epothilone conjugates of general formula I 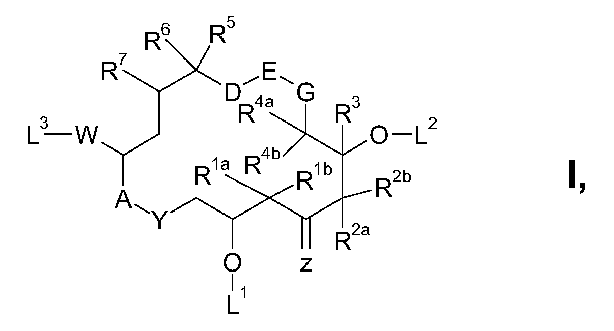 in which
- R^{1a}, R^{1b},: independently of one another, are hydrogen, C₁-C₁₀ alkyl, aryl, aralkyl, or together a -(CH₂)ₘ group, in which m is 2 to 5,
- R^{2a}, R^{2b},: independently of one another, are hydrogen, C₁-C₁₀ alkyl, aryl, aralkyl, or together a -(CH₂)ₙ group, in which n is 2 to 5, or C₂-C₁₀ alkenyl, or C₂-C₁₀ alkinyl,
- R³: is hydrogen, C₁-C₁₀ alkyl, aryl or aralkyl, and
- R^{4a}, R^{4b},: independently of one another, are hydrogen, C₁-C₁₀ alkyl, aryl, aralkyl, or together a -(CH₂)ₚ group, in which p is 2 to 5,
- R⁵: is hydrogen, C₁-C₁₀ alkyl, aryl, aralkyl, CO₂H, CO₂alkyl, CH₂OH, CH₂Oalkyl, CH₂Oacyl, CN, CH₂NH₂, CH₂N(alkyl, acyl)_{1,2}, or CH₂Hal,
- Hal: is a halogen atom,
- R⁶, R⁷,: in each case, are hydrogen, or together an additional bond or together an oxygen atom, or together an NH group, or together an N-alkyl group, or together a CH₂ group, and
- G: is an oxygen atom or CH₂,
- D-E: is a group H₂C-CH₂, HC=CH, C≡C, CH(OH)-CH(OH), CH(OH)-CH₂, CH₂-CH(OH), 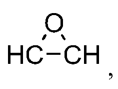 O-CH₂, or, if G represents a CH₂ group, is CH₂-O,
- W: is a group C(=X)R⁸, or a bi- or tricyclic aromatic or heteroaromatic radical,
- L³: is hydrogen, or, if a radical in W contains a hydroxyl group, forms a group O-L⁴ with the latter, or, if a radical in W contains an amino group, forms a group NR²⁵-L⁴ with the latter,
- R²⁵: is hydrogen or C₁-C₁₀ alkyl,
- X: is an oxygen atom, or two OR²⁰ groups, or a C₂-C₁₀ alkylenedioxy group, which should be straight-chain or branched, or H/OR⁹, or a CR¹⁰R¹¹ group,
- R⁸: is hydrogen, C₁-C₁₀ alkyl, aryl, aralkyl, halogen or CN, and
- R⁹: is hydrogen or a protective group PG^{X},
- R¹⁰, R¹¹,: in each case independently of one another, are hydrogen, C₁-C₂₀ alkyl, aryl, or aralkyl, or together with a methylene carbon atom form a 5- to 7-membered carbocyclic ring,
- Z: can represent oxygen or H/OR¹²,
- R¹²: can represent hydrogen or a protective group PG^{Z},
- A-Y: can represent a group O-C(=O), O-CH₂, CH₂-C(=O), NR²¹-C(=O) or NR²¹-SO₂,
- R²⁰: can represent C₁-C₂₀ alkyl,
- R²¹: can represent a hydrogen atom or C₁-C₁₀ alkyl,
- PG^{X},: PG^{Y}, and PG^{Z} can represent a protective group PG, and
L¹, L², L⁴, independently of one another, can represent hydrogen, a group C(=O)Cl, a group C(=S)Cl, a group PG^{Y} or a linker-recognition unit of general formula III;
with the condition that at least one substituent L¹, L² or L⁴ represents a linker-recognition unit of general formula (III) or a linker of general formula V; with the condition that at least one substituent L¹, L² or L⁴ represents a linker-recognition unit of general formula (III) or a linker of general formula (V)
the linker-recognition unit of general formula (III) has the following structure, 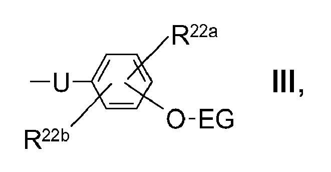 in which
- R^{22a}, R^{22b},: independently of one another, can represent hydrogen, C₁-C₂₀ alkyl, C₁-C₂₀ acyl, C₁-C₂₀ acyloxy, aryl, aralkyl, hydroxy, alkoxy, CO₂H, CO₂alkyl, halogen, CN, NO₂, NH₂, or N₃,
- U: can represent -C(=O)NR²³-, -C(=S)NR²³-, -C(=O)NR²³-CH₂-, -C(=S)NR²³-CH₂-, -C(=O)O-, -C(=S)O-, -C(=O)O-CH₂-, -C(=S)O-CH₂-,
- R²³: can represent hydrogen or C₁-C₁₀ alkyl, and
EG is a recognition unit of general formula IV, 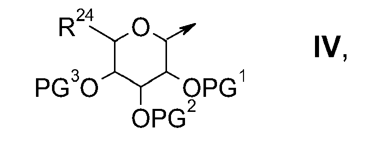 in which
- R²⁴: can represent a group CH₂OPG⁴ or a group CO₂R²⁶,
- PG¹, PG², PG³, and PG⁴,: independently of one another, can represent hydrogen or a protective group PG,
- R²⁶: can represent hydrogen, C₁-C₂₀ alkyl, C₁-C₂₀ alkenyl, C₄-C₇ cycloalkyl, which can contain an oxygen atom, aryl, aralkyl, tris(C₁-C₂₀ alkyl)silyl, bis(C₁-C₂₀ alkyl)-arylsilyl, (C₁-C₂₀ alkyl)-diarylsilyl, or tris(aralkyl)-silyl,
- PG^{X}, PG^{Y}, and PG^{Z}: can represent a protective group PG,
the linker unit of general formula (V) has the following structure, 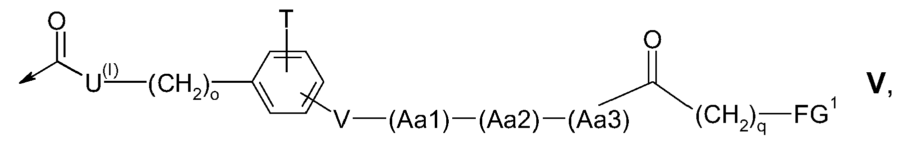 in which
- T: represents halogen, OH, O-alkyl, CO₂H CO₂-alkyl, -NHR^{23a}, -NO₂, -N₃, -CN, C₁-C₂₀-alkyl, C₁-C₂₀-acyl or C₁-C₂₀-acyloxy groups,
- U^{(I)}: represents a bond, oxygen, or NR^{24a},
- o: represents 0 to 5,
- V: represents oxygen or NR^{24b},
- Aa1: represents a bond or a group of general formula VI and
- Aa2, Aa3,: independently of one another, represent a group of general formula 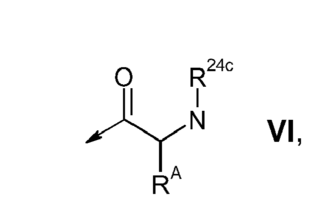 which is derived from a natural or unnatural amino acid HO-Aal-H, HO-Aa2-H, or HO-Aa3-H of general formula VI' 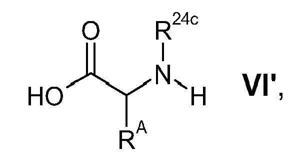 in which R^{A} can be the same or different in HO-Aal-H, HO-Aa2-H, or HO-Aa3-VI H, and the a-substituent represents a natural or unnatural amino acid,
- R²²: can represent hydrogen, C₁-C₁₀ alkyl, aryl or aralkyl,
- R^{23a}: can represent hydrogen, C₁-C₁₀ alkyl, or C₁-C₁₀ acyl,
- R^{24a}, R^{24b}, R^{24c},: independently of one another, can represent hydrogen or C₁-C₁₀ alkyl,
- q: can represent 1 to 20,
- FG¹: can represent C₁-C₁₀ alkyl-S₃, 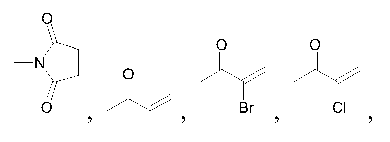 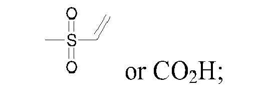
as a uniform isomer or a mixture of different isomers and/or as a pharmaceutically acceptable salt thereof.

The following compounds mentioned below are especially preferred as effector elements in the epothilones of the present invention:
(4S,7R,8S,9S,13Z,16S(E))-4,8-Dihydroxy-5,5,7,9,13-pentamethyl-16-[1-methyl-2-(2-methyl-thiazol-4-yl)-vinyl]-oxacyclohexadec-13-ene-2,6-dione;
(4S,7R,8S,9S,13Z,16S(E))-4,8-Dihydroxy-16-[2-(2-hydroxymethyl-thiazo1-4-yl)-1-methyl-vinyl]-5,5,7,9,13-pentamethyl-oxacyclohexadec-13-ene-2,6-dione;
(4S,7R,8S,9S,13Z,16S(E))-16-[2-(2-Aminomethyl-thiazo1-4-yl)-1-methyl-vinyl]-4,8-dihydroxy-5,5,7,9,13-pentamethyl-oxacyclohexadec-13-ene-2,6-dione;
(1S,3S(E),7S,10R,11S,12S,16R)-7,11-Dihydroxy-8,8,10,12,16-pentamethyl-3-[1-methyl-2-(2-methyl-thiazol-4-yl)-vinyl]-4,17-dioxa-bicyclo[14.1.0]heptadecane-5,9-dione;
(1S,3S(E),7S,10R,11S,12S,16R)-7,11-Dihydroxy-3-[2-(2-hydroxymethyl-thiazol-4-yl)-1-methyl-vinyl]-8,8,10,12,16-pentamethyl-4,17-dioxa-bicyclo[14.1.0]-heptadecane-5,9-dione;
(1S,3S(E),7S,10R,11S,12S,16R)-3-[2-(2-Aminomethyl-thiazo1-4-yl)-1-methyl-vinyl]-7,11-dihydroxy-8,8,10,12,16-pentamethyl-4,17-dioxa-bicyclo[14.1.0]-heptadecane-5,9-dione;
(4S,7R,8S,9S,13Z,16S(E))-4,8-Dihydroxy-7-ethyl-5,5,9,13-tetramethyl-16-[1-methyl-2-(2-methyl-thiazol-4-yl)-vinyl]-oxacyclohexadec-13-ene-2,6-dione;
(4S,7R,8S,9S,13Z,16S(E)-)-4,8-Dihydroxy-16-[2-(2-hydroxymethyl-thiazol-4-yl)-1 methyl-vinyl]-7-ethyl-5,5,9,13-tetramethyl-oxacyclohexadec-13-ene-2,6-dione;
(4S,7R,8S,9S,13Z,16S(E))-16-[2-(2-Aminomethyl-thiazol-4-yl)-1-methyl-vinyl]-4,8-dihydroxy-7-ethyl-5,5,9,13-tetramethyl-oxacyclohexadec-13-ene-2,6-dione;
(1S,3S(E),75,10R,11S,12S,16R)-7,11-Dihydroxy-10-ethyl-8,8,12,16-tetramethyl-3-[1-methyl-2-(2-methyl-thiazol-4-yl)-vinyl]-4,17-dioxa-bicyclo[14.1.0]-heptadecane-5,9-dione;
(1S,3S(E),7S,10R,11S,12S,16R)-7,11-Dihydroxy-3-[2-(2-hydroxymethyl-thiazol-4-yl)-1-methyl-vinyl]-10-ethyl-8,8,12,16-tetramethyl-4,17-dioxa-bicyclo[14.1.0]heptadecane-5,9-dione;
(1S,3S(E),7S,110R,1S,12S,16R)-3-[2-(2-Aminomethyl-thiazo1-4-yl)-1-methyl-vinyl]-7,11-dihydroxy-10-ethyl-8,8,12,16-tetramethyl-4,17-dioxa-bicyclo[14.1.0]-heptadecane-5,9-dione;
(4S,7R,8S,9S,13Z,16S(Z))-4,8-Dihydroxy-5,5,7,9,13-pentamethyl-16-[1-fluoro-2-(2-methyl-thiazol-4-yl)-vinyl]-oxacyclohexadec-13-ene-2,6-dione;
(4S,7R,8S,9S,13Z,16S(Z))-4,8-Dihydroxy-16-[2-(2-hydroxymethyl-thiazol-4-yl)-1-fluorovinyl]-5,5,7,9,13-pentamethyl-oxacyclohexadec-13-ene-2,6-dione;
(4S,7R,8S,9S,13Z,16S(Z))-16-[2-(2-Aminomethyl-thiazol-4-yl)-1-fluoro-vinyl]-4,8-dihydroxy-5,5,7,9,13-pentamethyl-oxacyclohexadec-13-ene-2,6-dione;
(1S,3S(Z),7S,10R,11S,12S,16R)-7,11-Dihydroxy-8,8,10,12,16-pentamethyl-3-[1-fluoro-2-(2-methyl-thiazol-4-yl)-vinyl]-4,17-dioxa-bicyclo[14.1.0]heptadecane-5,9-dione;
(1S,3S(Z),7S,110R,11S,12S,16R)-7,11-Dihydroxy-3-[2-(2-hydroxymethyl-thiazol-4-yl)-1-fluoro-vinyl]-8,8,10,12,16-pentamethyl-4,17-dioxa-bicyclo[14.1.0]-heptadecane-5,9-dione;
(1S,3S(Z),7S,10R,11S,12S,16R)-3-[2-(2-Aminomethyl-thiazol-4-yl)-1-fluoro-vinyl]-7,11-dihydroxy-8,8,10,12,16-pentamethyl-4,17-dioxa-bicyclo[14.1.0]-heptadecane-5,9-dione;
(4S,7R,8S,9S,13Z,16S(Z))-4,8-Dihydroxy-5,5,7,9,13-pentamethyl-16-[1-chloro-2-(2-methyl-thiazol-4-yl)-vinyl]-oxacyclohexadec-13-ene-2,6-dione;
(4S,7R,8S,9S,13Z,16S(Z))-4,8-Dihydroxy-16-[2-(2-hydroxymethyl-thiazol-4-yl)-1-chloro-vinyl]-5,5,7,9,13-pentamethyl-oxacyclohexadec-13-ene-2,6-dione;
(4S,7R,8S,9S,13Z,16S(Z))-16-[2-(2-Aminomethyl-thiazo1-4-yl)-1-chloro-vinyl]-4,8-dihydroxy-5,5,7,9,13-pentamethyl-oxacyclohexadec-13-ene-2,6-dione;
(1S,3S(Z),75,110R,11S,12S,16R)-7,11-Dihydroxy-8,8,10,12,16-pentamethyl-3-[1-chloro-2-(2-methyl-thiazol-4-yl)-vinyl]-4,17-dioxa-bicyclo[14.1.0]heptadecane-5,9-dione;
(1S,3S(Z),7S,10R,11 S,12S,16R)-7,11-Dihydroxy-3-[2-(2-hydroxymethyl-thiazol-4-yl)-1-chloro-vinyl]-8,8,10,12,16-pentamethyl-4,17-dioxa-bicyclo[14.1.0]-heptadecane-5,9-dione;
(1S,3S(Z),7S,10R,11S,12S,16R)-3-[2-(2-Aminomethyl-thiazol-4-yl)-1-chloro-vinyl]-7,11-dihydroxy-8,8,10,12,16-pentamethyl-4,17-dioxa-bicyclo[14.1.0]-heptadecane-5,9-dione;
(4S,7R,8S,9S,13Z,16S(Z))-4,8-Dihydroxy-7-ethyl-5,5,9,13-tetramethyl-16-[1-fluoro-2-(2-methyl-thiazol-4-yl)-vinyl]-oxacyclohexadec-13-ene-2,6-dione;
(4S,7R,8S,9S,13Z,16S(Z))-4,8-Dihydroxy-16-[2-(2-hydroxymethyl-thiazol-4-yl)-1-fluorovinyl]-7-ethyl-5,5,9,13-tetramethyl-oxacyclohexadec-13-ene-2,6-dione;
(4S,7R,8S,9S,13Z,16S(Z))-16-[2-(2-Aminomethyl-thiazol-4-yl)-1-fluoro-vinyl]-4,8-dihydroxy-7-ethyl-5,5,9,13-tetramethyl-oxacyclohexadec-13-ene-2,6-dione;
(1S,3S(Z),7S,10R,11S,12S,16R)-7,11-Dihydroxy-10-ethyl-8,8,12,16-tetramethyl-3-[1-fluoro-2-(2-methyl-thiazol-4-yl)-vinyl]-4,17-dioxa-bicyclo [14.1.0]-heptadecane-5,9-dione;
(1S,3S(Z),7S,10R,11S,12S,16R)-7,11-Dihydroxy-3-[2-(2-hydroxymethyl-thiazol-4-yl)-1-fluoro-vinyl]-10-ethyl-8,8,12,16-tetramethyl-4,17-dioxa-bicyclo[14.1.0]-heptadecane-5,9-dione;
(1S,3S(Z),7S,10R,11S,12S,16R)-3-[2-(2-Aminomethyl-thiazol-4-yl)-1-fluoro-vinyl]-7,11-dihydroxy-10-ethyl-8,8,12,16-tetramethyl-4,17-dioxa-bicyclo[14.1.0]-heptadecane-5,9-dione;
(4S,7R,8S,9S,13Z,16S(Z))-4,8-Dihydroxy-7-ethyl-5,5,9,13-tetramethyl-16-[1-chloro-2-(2-methyl-thiazol-4-yl)-vinyl]-oxacyclohexadec-13-ene-2,6-dione;
(4S,7R,8S,9S,13Z,16S(Z))-4,8-Dihydroxy-16-[2-(2-hydroxymethyl-thiazol-4-yl)-1-chloro-vinyl]-7-ethyl-5,5,9,13-tetramethyl-oxacyclohexadec-13-ene-2,6-dione;
(4S,7R,8S,9S,13Z,16S(Z))-16-[2-(2-Aminomethyl-thiazo1-4-yl)-1-chloro-vinyl]-4,8-dihydroxy-7-ethyl-5,5,9,13-tetramethyl-oxacyclohexadec-13-ene-2,6-dione;
(1S,3S(Z),7S,10R,1S,12S,16R)-7,11-Dihydroxy-10-ethyl-8,8,12,16-tetramethyl-3-[1-chloro-2-(2-methyl-thiazol-4-yl)-vinyl]-4,17-dioxa-bicyclo[14.1.0]-heptadecane-5,9-dione;
(1S,3S(Z),7S,10R,11S,12S,16R)-7,11-Dihydroxy-3-[2-(2-hydroxymethyl-thiazol-4-yl)-1-chloro-vinyl]-10-ethyl-8,8,12,16-tetramethyl-4,17-dioxa-bicyclo[14.1.0]heptadecane-5,9-dione;
(1S,3S(Z),7S,110R,1S,12S,16R)-3-[2-(2-Aminomethyl-thiazol-4-yl)-1-chloro-vinyl]-7,11-dihydroxy-10-ethyl-8,8,12,16-tetramethyl-4,17-dioxa-bicyclo[14.1.0]-heptadecane-5,9-dione;
(4S,7R,8S,9S,13Z,16S(E))-4,8-Dihydroxy-5,5,7,9,13-pentamethyl-16-[1-methyl-2-(2-pyridyl)-vinyl]-oxacyclohexadec-13-ene-2,6-dione;
(1S,3S(E),75,10R,11S,12S,16R)-7,11-Dihydroxy-8,8,10,12,16-pentamethyl-3-[1-methyl-2-(2-pyridyl)-vinyl]-4,17-dioxa-bicyclo[14.1.0]heptadecane-5,9-dione;
(4S,7R,8S,9S,13Z,16S(E))-4,8-Dihydroxy-7-ethyl-5,5,9,13-tetramethyl-16-[1-methyl-2-(2-pyridyl)-vinyl]-oxacyclohexadec-13-ene-2,6-dione;
(1S,3S(E),75,10R,11S,12S,16R)-7,11-Dihydroxy-10-ethyl-8,8,12,16-tetramethyl-3-[1-methyl-2-(2-pyridyl)-vinyl]-4,17-dioxa-bicyclo[14.1.0]heptadecane-5,9-dione;
(4S,7R,8S,9S,13Z,16S(Z))-4,8-Dihydroxy-5,5,7,9,13-pentamethyl-16-[1-fluoro-2-(2-pyridyl)-vinyl]-oxacyclohexadec-13-ene-2,6-dione;
(1 S,3S(Z),7S, 10R,11S,12S,16R)-7,11-Dihydroxy-8,8,10,12,16-pentamethyl-3-[1-fluoro-2-(2-pyridyl)-vinyl]-4,17-dioxa-bicyclo [14.1.0]heptadecane-5,9-dione;
(4S,7R,8S,9S,13Z,16S(Z))-4,8-Dihydroxy-5,5,7,9,13-pentamethyl-16-[1-chloro-2-(2-pyridyl)-vinyl]-oxacyclohexadec-13-ene-2,6-dione;
(1 S,3S(Z),7S,10R,11S,12S,16R)-7,11-Dihydroxy-8,8,10,12,16-pentamethyl-3-[1-chloro-2-(2-pyridyl)-vinyl]-4,17-dioxa-bicyclo [14.1.0]heptadecane-5,9-dione;
(4S,7R,8S,9S,13Z,16S(Z))-4,8-Dihydroxy-7-ethyl-5,5,9,13-tetramethyl-16-[1-fluoro-2-(2-pyridyl)-vinyl]-oxacyclohexadec-13-ene-2,6-dione;
(1S,3S(Z),75,10R,11S,125,16R)-7,11-Dihydroxy-10-ethyl-8,8,12,16-tetramethyl-3-[1-fluoro-2-(2-pyridyl)-vinyl]-4,17-dioxa-bicyclo [14.1.0]heptadecane-5,9-dione;
(4S,7R,8S,9S,13Z,16S(Z))-4,8-Dihydroxy-7-ethyl-5,5,9,13-tetramethyl-16-[1-chloro-2-(2-pyridyl)-vinyl]-oxacyclohexadec-13-ene-2,6-dione;
(1S,3S(Z),75,110R,1S,12S,16R)-7,11-Dihydroxy-10-ethyl-8,8,12,16-tetramethyl-3-[1-chloro-2-(2-pyridyl)-vinyl]-4,17-dioxa-bicyclo[14.1.0]heptadecane-5,9-dione;
(4S,7R,8S,9S,13Z,16S(E))-4,8-Dihydroxy-5,5,7,9,13-pentamethyl-16-[1-methyl-2-(2-methyl-oxazol-4-yl)-vinyl]-oxacyclohexadec-13-ene-2,6-dione;
(4S,7R,8S,9S,13Z,16S(E))-4,8-Dihydroxy-16-[2-(2-hydroxymethyl-oxazol-4-yl)-1-methylvinyl]-5,5,7,9,13-pentamethyl-oxacyclohexadec-13-ene-2,6-dione;
(4S,7R,8S,9S,13Z,16S(E)-)-16-[2-(2-Aminomethyl-oxazol-4-yl)-1-methyl-vinyl]-4,8 dihydroxy-5,5,7,9,13-pentamethyl-oxacyclohexadec-13-ene-2,6-dione;
(1S,3S(E),75,10R,11S,12S,16R)-7,11-Dihydroxy-8,8,10,12,16-pentamethyl-3-[1-methyl-2-(2-methyl-oxazol-4-yl)-vinyl]-4,17-dioxa-bicyclo[14.1.0]heptadecane-5,9-dione;
(1S,3S(E),7S,10R,11S,12S,16R)-7,11-Dihydroxy-3-[2-(2-hydroxymethyl-oxazol-4-yl)-1-methyl-vinyl]-8,8,10,12,16-pentamethyl-4,17-dioxa-bicyclo[14.1.0]-heptadecane-5,9-dione;
(1S,3S(E),7S,10R,11S,12S,16R)-3-[2-(2-Aminomethyl-oxazol-4-yl)-1-methyl-vinyl]-7,11-dihydroxy-8,8,10,12,16-pentamethyl-4,17-dioxa-bicyclo[14.1.0]-heptadecane-5,9-dione;
(4S,7R,8S,9S,13Z,16S(E))-4,8-Dihydroxy-7-ethyl-5,5,9,13-tetramethyl-16-[1-methyl-2-(2-methyl-oxazol-4-yl)-vinyl]-oxacyclohexadec-13-ene-2,6-dione;
(4S,7R,8S,9S,13Z,16S(E))-4,8-Dihydroxy-16-[2-(2-hydroxymethyl-oxazol-4-yl)-1-methylvinyl]-7-ethyl-5,5,9,13-tetramethyl-oxacyclohexadec-13-ene-2,6-dione;
(4S,7R,8S,9S,13Z,16S(E)-)-16-[2-(2-Aminomethyl-oxazol-4-yl)-1-methyl-vinyl]-4,8 dihydroxy-7-ethyl-5,5,9,13-tetramethyl-oxacyclohexadec-13-ene-2,6-dione;
(1S,3S(E),7S,10R,11S,12S,16R)-7,11-Dihydroxy-10-ethyl-8,8,12,16-tetramethyl-3-[1-methyl-2-(2-methyl-oxazol-4-yl)-vinyl]-4,17-dioxa-bicyclo[14.1.0]-heptadecane-5,9-dione;
(1S,3S(E),7S,10R,11S,12S,16R)-7,11-Dihydroxy-3-[2-(2-hydroxymethyl-oxazol-4-yl)-1-methyl-vinyl]-10-ethyl-8,8,12,16-tetramethyl-4,17-dioxa-bicyclo[14.1.0]heptadecane-5,9-dione;
(1S,3S(E),7S,10R,11S,12S,16R)-3-[2-(2-Aminomethyl-oxazo1-4-yl)-1-methyl-vinyl]-7,11-dihydroxy-10-ethyl-8,8,12,16-tetramethyl-4,17-dioxa-bicyclo[14.1.0]-heptadecane-5,9-dione;
(4S,7R,8S,9S,13Z,16S(Z))-4,8-Dihydroxy-5,5,7,9,13-pentamethyl-16-[1-fluoro-2-(2-methyl-oxazol-4-yl)-vinyl]-oxacyclohexadec-13-ene-2,6-dione;
(4S,7R,8S,9S,13Z,16S(Z))-4,8-Dihydroxy-16-[2-(2-hydroxymethyl-oxazol-4-yl)-1-fluorovinyl]-5,5,7,9,13-pentamethyl-oxacyclohexadec-13-ene-2,6-dione;
(4S,7R,8S,9S,13Z,16S(Z))-16-[2-(2-Aminomethyl-oxazol-4-yl)-1-fluoro-vinyl]-4,8-dihydroxy-5,5,7,9,13-pentamethyl-oxacyclohexadec-13-ene-2,6-dione;
(1 S,3S(Z),7S, 10R,11S,12S,16R)-7,11-Dihydroxy-8,8,10,12,16-pentamethyl-3-[1-fluoro-2-(2-methyl-oxazol-4-yl)-vinyl]-4,17-dioxa-bicyclo [14.1.0]heptadecane-5,9-dione;
(1S,3S(Z),7S,10R,11S,12S,16R)-7,11-Dihydroxy-3-[2-(2-hydroxymethyl-oxazol-4-yl)-1-fluoro-vinyl]-8,8,10,12,16-pentamethyl-4,17-dioxa-bicyclo[14.1.0]-heptadecane-5,9-dione;
(1S,3S(Z),7S,10R,11S,12S,16R)-3-[2-(2-Aminomethyl-oxazol-4-yl)-1-fluoro-vinyl]-7,11-dihydroxy-8,8,10,12,16-pentamethyl-4,17-dioxa-bicyclo[14.1.0]-heptadecane-5,9-dione;
(4S,7R,8S,9S,13Z,16S(Z))-4,8-Dihydroxy-5,5,7,9,13-pentamethyl-16-[1-chloro-2-(2-methyl-oxazol-4-yl)-vinyl]-oxacyclohexadec-13-ene-2,6-dione;
(4S,7R,8S,9S,13Z,16S(Z))-4,8-Dihydroxy-16-[2-(2-hydroxymethyl-oxazol-4-yl)-1-chloro-vinyl]-5,5,7,9,13-pentamethyl-oxacyclohexadec-13-ene-2,6-dione;
(4S,7R,8S,9S,13Z,16S(Z)-)-16-[2-(2-Aminomethyl-oxazol-4-yl)-1-chloro-vinyl]-4,8 dihydroxy-5,5,7,9,13-pentamethyl-oxacyclohexadec-13-ene-2,6-dione;
(1S,3S(Z),7S,10R,11S,12S,16R)-7,11-Dihydroxy-8,8,10,12,16-pentamethyl-3-[1-chloro-2-(2-methyl-oxazol-4-yl)-vinyl]-4,17-dioxa-bicyclo[14.1.0]heptadecane-5,9-dione;
(1S,3S(Z),7S,10R,11S,12S,16R)-7,11-Dihydroxy-3-[2-(2-hydroxymethyl-oxazol-4-yl)-1-chloro-vinyl]-8,8,10,12,16-pentamethyl-4,17-dioxa-bicyclo[14.1.0]-heptadecane-5,9-dione;
(1S,3S(Z),7S,10R,11S,12S,16R)-3-[2-(2-Aminomethyl-oxazol-4-yl)-1-chloro-viny1]-7,11-dihydroxy-8,8,10,12,16-pentamethyl-4,17-dioxa-bicyclo[14.1.0]-heptadecane-5,9-dione;
(4S,7R,8S,9S,13Z,16S(Z))-4,8-Dihydroxy-7-ethyl-5,5,9,13-tetramethyl-16-[1-fluoro-2-(2-methyl-oxazol-4-yl)-vinyl]-oxacyclohexadec-13-ene-2,6-dione;
(4S,7R,8S,9S,13Z,16S(Z))-4,8-Dihydroxy-16-[2-(2-hydroxymethyl-oxazol-4-yl)-1-fluorovinyl]-7-ethyl-5,5,9,13-tetramethyl-oxacyclohexadec-13-ene-2,6-dione;
(4S,7R,8S,9S,13Z,16S(Z))-16-[2-(2-Aminomethyl-oxazol-4-yl)-1-fluoro-vinyl]-4,8-dihydroxy-7-ethyl-5,5,9,13-tetramethyl-oxacyclohexadec-13-ene-2,6-dione;
(1S,3S(Z),7S,10R,11S,12S,16R)-7,11-Dihydroxy-10-ethyl-8,8,12,16-tetramethyl-3-[1-fluoro-2-(2-methyl-oxazol-4-yl)-vinyl]-4,17-dioxa-bicyclo[14.1.0]-heptadecane-5,9-dione;
(1S,3S(Z),75,110R,1S,12S,16R)-7,11-Dihydroxy-3-[2-(2-hydroxymethyl-oxazol-4-yl)-1-fluoro-vinyl]-10-ethyl-8,8,12,16-tetramethyl-4,17-dioxa-bicyclo[14.1.0]heptadecane-5,9-dione;
(1S,3S(Z),7S,10R,11S,12S,16R)-3-[2-(2-Aminomethyl-oxazol-4-yl)-1-fluoro-vinyl]-7,11-dihydroxy-10-ethyl-8,8,12,16-tetramethyl-4,17-dioxa-bicyclo[14.1.0]-heptadecane-5,9-dione;
(4S,7R,8S,9S,13Z,16S(Z))-4,8-Dihydroxy-7-ethyl-5,5,9,13-tetramethyl-16-[1-chloro-2-(2-methyl-oxazol-4-yl)-vinyl]-oxacyclohexadec-13-ene-2,6-dione;
(4S,7R,8S,9S,13Z,16S(Z))-4,8-Dihydroxy-16-[2-(2-hydroxymethyl-oxazol-4-yl)-1-chloro-vinyl]-7-ethyl-5,5,9,13-tetramethyl-oxacyclohexadec-13-ene-2,6-dione;
(4S,7R,8S,9S,13Z,16S(Z))-16-[2-(2-Aminomethyl-oxazol-4-yl)-1-chloro-vinyl]-4,8 dihydroxy-7-ethyl-5,5,9,13-tetramethyl-oxacyclohexadec-13-ene-2,6-dione;
(1S,3S(Z),75,10R,11S,12S,16R)-7,11-Dihydroxy-10-ethyl-8,8,12,16-tetramethyl-3-[1-chloro-2-(2-methyl-oxazol-4-yl)-vinyl]-4,17-dioxa-bicyclo [14.1.0]-heptadecane-5,9-dione;
(1S,3S(Z),75,10R,11S,12S,16R)-7,11-Dihydroxy-3-[2-(2-hydroxymethyl-oxazol-4-yl)-1-chloro-vinyl]-10-ethyl-8,8,12,16-tetramethyl-4,17-dioxa-bicyclo[14.1.0]-heptadecane-5,9-dione;
(1S,3S(Z),7S,10R,11S,12S,16R)-3-[2-(2-Aminomethyl-oxazol-4-yl)-1-chloro-vinyl]-7,11-dihydroxy-10-ethyl-8,8,12,16-tetramethyl-4,17-dioxa-bicyclo[14.1.0]-heptadecane-5,9-dione;
(4S,7R,8S,9S,13Z,16S(E))-4,8-Dihydroxy-5,5,7,9,13-pentamethyl-16-[2-(2-methyl-thiazol-4-yl)-vinyl]-oxacyclohexadec-13-ene-2, 6-dione;
(4S,7R,8S,9S,13Z,16S(E))-4,8-Dihydroxy-16-[2-(2-hydroxymethyl-thiazol-4-yl)-vinyl]-5,5,7,9,13-pentamethyl-oxacyclohexadec-13-ene-2,6-dione;
(4S,7R,8S,9S,13Z,16S(E))-16-[2-(2-Aminomethyl-thiazol-4-yl)-vinyl]-4,8-dihydroxy-5,5,7,9,13-pentamethyl-oxacyclohexadec-13-ene-2,6-dione;
(1S,3S(E),75,110R,11S,12S,16R)-7,11-Dihydroxy-8,8,10,12,16-pentamethyl-3-[2-(2-methyl-thiazol-4-yl)-vinyl]-4,17-dioxa-bicyclo[14.1.0]heptadecane-5,9-dione;
(1S,3S(E),7S,110R,11S,12S,16R)-7,11-Dihydroxy-3-[2-(2-hydroxymethyl-thiazol-4-yl)-vinyl]-8,8,10,12,16-pentamethyl-4,17-dioxa-bicyclo[14.1.0]heptadecane-5,9-dione;
(1S,3S(E),75,10R,11S,12S,16R)-3-[2-(2-Aminomethyl-thiazol-4-yl)-vinyl]-7,11-dihydroxy-8,8,10,12,16-pentamethyl-4,17-dioxa-bicyclo[14.1.0]heptadecane-5,9-dione;
(4S,7R,8S,9S,13Z,16S(E))-4,8-Dihydroxy-7-ethyl-5,5,9,13-tetramethyl-16-[2-(2-methyl-thiazo1-4-yl)-vinyl]-oxacyclohexadec-13-ene-2,6-dione;
(4S,7R,8S,9S,13Z,16S(E))-4,8-Dihydroxy-16-[2-(2-hydroxymethyl-thiazo1-4-yl)-vinyl]-7-ethyl-5,5,9,13-tetramethyl-oxacyclohexadec-13-ene-2,6-dione;
(4S,7R,8S,9S,13Z,16S(E))-16-[2-(2-Aminomethyl-thiazo1-4-yl)-vinyl]-4,8-dihydroxy-7-ethyl-5,5,9,13-tetramethyl-oxacyclohexadec-13-ene-2,6-dione;
(1 S,3S(E),7S, 10R,11S,12S,16R)-7,11-Dihydroxy-10-ethyl-8,8,12,16-tetramethyl-3-[2-(2-methyl-thiazol-4-yl)-vinyl]-4,17-dioxa-bicyclo[14.1.0]heptadecane-5,9-dione;
(1S,3S(E),7S,10R,11S,12S,16R)-7,11-Dihydroxy-3-[2-(2-hydroxymethyl-thiazol-4-yl)-vinyl]-10-ethyl-8,8,12,16-tetramethyl-4,17-dioxa-bicyclo[14.1.0]-heptadecane-5,9-dione;
(1S,3S(E),7S, 10R,111S,125,16R)-3-[2-(2-Aminomethyl-thiazol-4-yl)-vinyl]-7,11-dihydroxy-10-ethyl-8,8,12,16-tetramethyl-4,17-dioxa-bicyclo[14.1.0]heptadecane-5,9-dione;
(4S,7R,8S,9S,13Z,16S(E))-4,8-Dihydroxy-5,5,7,9,13-pentamethyl-16-[2-(2-pyridyl)-vinyl]-oxacyclohexadec-13-ene-2,6-dione;
(1S,3S(E),75,10R,11S,12S,16R)-7,11-Dihydroxy-8,8,10,12,16-pentamethyl-3-[2-(2-pyridyl)-vinyl]-4,17-dioxa-bicyclo[14.1.0]heptadecane-5,9-dione;
(4S,7R,8S,9S,13Z,16S(E))-4,8-Dihydroxy-7-ethyl-5,5,9,13-tetramethyl-16-[2-(2-pyridyl)-vinyl]-oxacyclohexadec-13-ene-2,6-dione;
(1 S,3S(E),7S, 10R,11S,12S,16R)-7,11-Dihydroxy-10-ethyl-8,8,12,16-tetramethyl-3-[2-(2-pyridyl)-vinyl]-4,17-dioxa-bicyclo[14.1.0]heptadecane-5,9-dione;
(4S,7R,8S,9S,13Z,16S)-4,8-Dihydroxy-5,5,7,9,13-pentamethyl-16-(2-methyl-benzothiazol-5-yl)-oxacyclohexadec-13-ene-2,6-dione;
(4S,7R,8S,9S,13Z,16S)-4,8-Dihydroxy-16-(2-hydroxymethyl-benzothiazo1-5-yl)-5,5,7,9,13-pentamethyl-oxacyclohexadec-13-ene-2,6-dione;
(4S,7R,8S,9S,13Z,16S)-16-(2-Aminomethyl-benzothiazol-5-yl)-4,8-dihydroxy-5,5,7,9,13-pentamethyl-oxacyclohexadec-13-ene-2,6-dione;
(1 S,3S,7S,10R,11S,12S,16R)-7,11-Dihydroxy-8,8,10,12,16-pentamethyl-3-(2-methyl-benzothiazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzothiazol-5-yl)-8,8,10,12,16-pentamethyl-4,17-dioxa-bicyclo[14.1.0]heptadecane-5,9-dione;
(1S,3S,7S,10R,11S,12S,16R)-3-(2-Aminomethyl-benzothiazol-5-yl)-7,11-dihydroxy-8,8,10,12,16-pentamethyl-4,17-dioxa-bicyclo[14.1.0]heptadecane-5,9-dione;
(4S,7R,8S,9S,13Z,16S)-4,8-Dihydroxy-7-ethyl-5,5,9,13-tetramethyl-16-(2-methyl-benzothiazol-5-yl)-oxacyclohexadec-13-ene-2,6-dione;
(4S,7R,8S,9S,13Z,16S)-4,8-Dihydroxy-16-(2-hydroxymethyl-benzothiazo1-5-yl)-7-ethyl-5,5,9,13-tetramethyl-oxacyclohexadec-13-ene-2,6-dione;
(4S,7R,8S,9S,13Z,16S)-16-(2-Aminomethyl-benzothiazo1-5-yl)-4,8-dihydroxy-7-ethyl-5,5,9,13-tetramethyl-oxacyclohexadec-13-ene-2,6-dione;
(1S,3S,7S,10R,11S,12S,16R)-7,11-Dihydroxy-10-ethyl-8,8,12,16-tetramethyl-3-(2-methyl-benzothiazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzothiazol-5-yl)-10-ethyl-8,8,12,16-tetramethyl-4,17-dioxa-bicyclo[14.1.0]-heptadecane-5,9-dione;
(1S,3S,7S,10R,11S,12S,16R)-3-(2-Aminomethyl-benzothiazol-5-yl)-7,11-dihydroxy-10-ethyl-8,8,12,16-tetramethyl-4,17-dioxa-bicyclo[14.1.0]heptadecane-5,9-dione;
(4S,7R,8S,9S,13Z,16S)-4,8-Dihydroxy-7-propyl-5,5,9,13-tetramethyl-16-(2-methyl-benzothiazol-5-yl)-oxacyclohexadec-13-ene-2,6-dione;
(4S,7R,8S,9S,13Z,16S)-4,8-Dihydroxy-16-(2-hydroxymethyl-benzothiazo1-5-yl)-7-propyl-5,5,9,13-tetramethyl-oxacyclohexadec-13-ene-2,6-dione;
(4S,7R,8S,9S,13Z,16S)-16-(2-Aminomethyl-benzothiazo1-5-yl)-4,8-dihydroxy-7-propyl-5,5,9,13-tetramethyl-oxacyclohexadec-13-ene-2,6-dione;
(1S,3S,7S,10R,11S,12S,16R)-7,11-Dihydroxy-10-propyl-8,8,12,16-tetramethyl-3-(2-methyl-benzothiazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzothiazol-5-yl)-10-propyl-8,8,12,16-tetramethyl-4,17-dioxa-bicyclo[14.1.0]-heptadecane-5,9-dione;
(1S,3S,7S,10R,11S,125,16R)-3-(2-Aminomethyl-benzothiazol-5-yl)-7,11-dihydroxy-10-propyl-8,8,12,16-tetramethyl-4,17-dioxa-bicyclo[14.1.0]heptadecane-5,9-dione;
(4S,7R,8S,9S,13Z,16S)-4,8-Dihydroxy-7-butyl-5,5,9,13-tetramethyl-16-(2-methyl-benzothiazol-5-yl)-oxacyclohexadec-13-ene-2,6-dione;
(4S,7R,8S,9S,13Z,16S)-4,8-Dihydroxy-16-(2-hydroxymethyl-benzothiazo1-5-yl)-7-butyl-5,5,9,13-tetramethyl-oxacyclohexadec-13-ene-2,6-dione;
(4S,7R,8S,9S,13Z,16S)-16-(2-Aminomethyl-benzothiazo1-5-yl)-4,8-dihydroxy-7-butyl-5,5,9,13-tetramethyl-oxacyclohexadec-13-ene-2,6-dione;
(1S,3S,7S,10R,11S,12S,16R)-7,11-Dihydroxy-10-butyl-8,8,12,16-tetramethyl-3-(2-methyl-benzothiazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzothiazol-5-yl)-10-butyl-8,8,12,16-tetramethyl-4,17-dioxa-bicyclo[14.1.0]-heptadecane-5,9-dione;
(1S,3S,7S,110R,1S,12S,16R)-3-(2-Aminomethyl-benzothiazol-5-yl)-7,11-dihydroxy-10-butyl-8,8,12,16-tetramethyl-4,17-dioxa-bicyclo[14.1.0]heptadecane-5,9-dione;
(4S,7R,8S,9S,13Z,16S)-4,8-Dihydroxy-7-allyl-5,5,9,13-tetramethyl-16-(2-methyl-benzothiazol-5-yl)-oxacyclohexadec-13-ene-2,6-dione;
(4S,7R,8S,9S,13Z,16S)-4,8-Dihydroxy-16-(2-hydroxymethyl-benzothiazo1-5-yl)-7-allyl-5,5,9,13-tetramethyl-oxacyclohexadec-13-ene-2,6-dione;
(4S,7R,8S,9S,13Z,16S)-16-(2-Aminomethyl-benzothiazol-5-yl)-4,8-dihydroxy-7-allyl-5,5,9,13-tetramethyl-oxacyclohexadec-13-ene-2,6-dione;
(1S,3S,7S,10R,11S,12S,16R)-7,11-Dihydroxy-10-allyl-8,8,12,16-tetramethyl-3-(2-methyl-benzothiazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzothiazol-5-yl)-10-allyl-8,8,12,16-tetramethyl-4,17-dioxa-bicyclo[14.1.0]-heptadecane-5,9-dione;
(1S,3S,7S,10R,11S,12S,16R)-3-(2-Aminomethyl-benzothiazol-5-yl)-7,11-dihydroxy-10-allyl-8,8,12,16-tetramethyl-4,17-dioxa-bicyclo[14.1.0]heptadecane-5,9-dione;
(4S,7R,8S,9S,13Z,16S)-4,8-Dihydroxy-7-prop-2-inyl-5,5,9,13-tetramethyl-16-(2-methyl-benzothiazol-5-yl)-oxacyclohexadec-13-ene-2,6-dione;
(4S,7R,8S,9S,13Z,16S)-4,8-Dihydroxy-16-(2-hydroxymethyl-benzothiazol-5-yl)-7-prop-2-inyl-5,5,9,13-tetramethyl-oxacyclohexadec-13-ene-2,6-dione;
(4S,7R,8S,9S,13Z,16S)-16-(2-Aminomethyl-benzothiazol-5-yl)-4,8-dihydroxy-7-prop-2-inyl-5,5,9,13-tetramethyl-oxacyclohexadec-13-ene-2,6-dione;
(1S,3S,7S,10R,11S,12S,16R)-7,11-Dihydroxy-10-prop-2-inyl-8,8,12,16-tetramethyl-3-(2-methyl-benzothiazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzothiazol-5-yl)-10-prop-2-inyl-8,8,12,16-tetramethyl-4,17-dioxa-bicyclo[14.1.0]-heptadecane-5,9-dione;
(1S,3S,7S,10R,11S,12S,16R)-3-(2-Aminomethyl-benzothiazol-5-yl)-7,11-dihydroxy-10-prop-2-inyl-8,8,12,16-tetramethyl-4,17-dioxa-bicyclo[14.1.0]-heptadecane-5,9-dione;
(4S,7R,8S,9S,13Z,16S)-4,8-Dihydroxy-7-but-3-enyl-5,5,9,13-tetramethyl-16-(2-methyl-benzothiazol-5-yl)-oxacyclohexadec-13-ene-2,6-dione;
(4S,7R,8S,9S,13Z,16S)-4,8-Dihydroxy-16-(2-hydroxymethyl-benzothiazol-5-yl)-7-but-3-enyl-5,5,9,13-tetramethyl-oxacyclohexadec-13-ene-2,6-dione;
(4S,7R,8S,9S,13Z,16S)-16-(2-Aminomethyl-benzothiazol-5-yl)-4,8-dihydroxy-7-but-3-enyl-5,5,9,13-tetramethyl-oxacyclohexadec-13-ene-2,6-dione;
(1S,3S,7S,10R,11S,12S,16R)-7,11-Dihydroxy-10-but-3-enyl-8,8,12,16-tetramethyl-3-(2-methyl-benzothiazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzothiazol-5-yl)-10-but-3-enyl-8,8,12,16-tetramethyl-4,17-dioxa-bicyclo[14.1.0]-heptadecane-5,9-dione;
(1S,3S,7S,10R,11S,12S,16R)-3-(2-Aminomethyl-benzothiazol-5-yl)-7,11-dihydroxy-10-but-3-enyl-8,8,12,16-tetramethyl-4,17-dioxa-bicyclo[14.1.0]heptadecane-5,9-dione;
(4S,7R,8S,9S,13Z,16S)-4,8-Dihydroxy-7-but-3-inyl-5,5,9,13-tetramethyl-16-(2-methyl-benzothiazol-5-yl)-oxacyclohexadec-13-ene-2,6-dione;
(4S,7R,8S,9S,13Z,16S)-4,8-Dihydroxy-16-(2-hydroxymethyl-benzothiazol-5-yl)-7-but-3-inyl-5,5,9,13-tetramethyl-oxacyclohexadec-13-ene-2,6-dione;
(4S,7R,8S,9S,13Z,16S)-16-(2-Aminomethyl-benzothiazol-5-yl)-4,8-dihydroxy-7-but-3-inyl-5,5,9,13-tetramethyl-oxacyclohexadec-13-ene-2,6-dione;
(1S,3S,7S,10R,11S,12S,16R)-7,11-Dihydroxy-10-but-3-inyl-8,8,12,16-tetramethyl-3-(2-methyl-benzothiazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzothiazol-5-yl)-10-but-3-inyl-8,8,12,16-tetramethyl-4,17-dioxa-bicyclo[14.1.0]-heptadecane-5,9-dione;
(1S,3S,7S,10R,1S,12S,16R)-3-(2-Aminomethyl-benzothiazol-5-yl)-7,11-dihydroxy-10-but-3-inyl-8,8,12,16-tetramethyl-4,17-dioxa-bicyclo[14.1.0]heptadecane-5,9-dione;
(4S,7R,8S,9S,13Z,16S)-4,8-Dihydroxy-5,5,7,9,13-pentamethyl-16-(2-methyl-benzoxazol-5-yl)-oxacyclohexadec-13-ene-2,6-dione;
(4S,7R,8S,9S,13Z,16S)-4,8-Dihydroxy-16-(2-hydroxymethyl-benzoxazol-5-yl)-5,5,7,9,13-pentamethyl-oxacyclohexadec-13-ene-2,6-dione;
(4S,7R,8S,9S,13Z,16S)-16-(2-Aminomethyl-benzoxazol-5-yl)-4,8-dihydroxy-5,5,7,9,13-pentamethyl-oxacyclohexadec-13-ene-2,6-dione;
(1S,3S,7S,10R,11S,12S,16R)-7,11-Dihydroxy-8,8,10,12,16-pentamethyl-3-(2-methyl-benzoxazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzoxazol-5-yl)-8,8,10,12,16-pentamethyl-4,17-dioxa-bicyclo[14.1.0]heptadecane-5,9-dione;
(1S,3S,7S,10R,11S,125,16R)-3-(2-Aminomethyl-benzoxazol-5-yl)-7,11-dihydroxy-8,8,10,12,16-pentamethyl-4,17-dioxa-bicyclo[14.1.0]heptadecane-5,9-dione;
(4S,7R,8S,9S,13Z,16S)-4,8-Dihydroxy-7-ethyl-5,5,9,13-tetramethyl-16-(2-methyl-benzoxazol-5-yl)-oxacyclohexadec-13-ene-2,6-dione;
(4S,7R,8S,9S,13Z,16S)-4,8-Dihydroxy-16-(2-hydroxymethyl-benzoxazo1-5-yl)-7-ethyl-5,5,9,13-tetramethyl-oxacyclohexadec-13-ene-2,6-dione;
(4S,7R,8S,9S,13Z,16S)-16-(2-Aminomethyl-benzoxazol-5-yl)-4,8-dihydroxy-7-ethyl-5,5,9,13-tetramethyl-oxacyclohexadec-13-ene-2,6-dione;
(1S,3S,7S,10R,11S,12S,16R)-7,11-Dihydroxy-10-ethyl-8,8,12,16-tetramethyl-3-(2-methyl-benzoxazol-5-yl)-4,17-dioxa-bicyclo[14.1.0]heptadecane-5,9-dione;
(1S,3S,7S, 10R,11S,12S,16R)-7,11-Dihydroxy-3-(2-hydroxymethyl-benzoxazol-5-yl)-10-ethyl-8,8,12,16-tetramethyl-4,17-dioxa-bicyclo[14.1.0]heptadecane-5,9-dione;
(1S,3S,7S,10R,11S,125,16R)-3-(2-Aminomethyl-benzoxazol-5-yl)-7,11-dihydroxy-10-ethyl-8,8,12,16-tetramethyl-4,17-dioxa-bicyclo[14.1.0]heptadecane-5,9-dione;
(4S,7R,8S,9S,13Z,16S)-4,8-Dihydroxy-7-propyl-5,5,9,13-tetramethyl-16-(2-methyl-benzoxazol-5-yl)-oxacyclohexadec-13-ene-2,6-dione;
(4S,7R,8S,9S,13Z,16S)-4,8-Dihydroxy-16-(2-hydroxymethyl-benzoxazo1-5-yl)-7-propyl-5,5,9,13-tetramethyl-oxacyclohexadec-13-ene-2,6-dione;
(4S,7R,8S,9S,13Z,16S)-16-(2-Aminomethyl-benzoxazol-5-yl)-4,8-dihydroxy-7-propyl-5,5,9,13-tetramethyl-oxacyclohexadec-13-ene-2,6-dione;
(1S,3S,7S,10R,11S,12S,16R)-7,11-Dihydroxy-10-propyl-8,8,12,16-tetramethyl-3-(2-methyl-benzoxazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzoxazol-5-yl)-10-propyl-8,8,12,16-tetramethyl-4,17-dioxa-bicyclo[14.1.0]heptadecane-5,9-dione;
(1S,3S,7S,10R,11S,125,16R)-3-(2-Aminomethyl-benzoxazol-5-yl)-7,11-dihydroxy-10-propyl-8,8,12,16-tetramethyl-4,17-dioxa-bicyclo[14.1.0]heptadecane-5,9-dione;
(4S,7R,8S,9S,13Z,16S)-4,8-Dihydroxy-7-butyl-5,5,9,13-tetramethyl-16-(2-methyl-benzoxazol-5-yl)-oxacyclohexadec-13-ene-2,6-dione;
(4S,7R,8S,9S,13Z,16S)-4,8-Dihydroxy-16-(2-hydroxymethyl-benzoxazo1-5-yl)-7-butyl-5,5,9,13-tetramethyl-oxacyclohexadec-13-ene-2,6-dione;
(4S,7R,8S,9S,13Z,16S)-16-(2-Aminomethyl-benzoxazol-5-yl)-4,8-dihydroxy-7-butyl-5,5,9,13-tetramethyl-oxacyclohexadec-13-ene-2,6-dione;
(1 S,3 S,7S,10R,11S,12S,16R)-7,11-Dihydroxy-10-butyl-8,8,12,16-tetramethyl-3-(2-methyl-benzoxazol-5-yl)-4,17-dioxa-bicyclo[14.1.0]heptadecane-5,9-dione;
(1 S,3S,7S,10R,11S,12S,16R)-7,11-Dihydroxy-3-(2-hydroxymethyl-benzoxazol-5-yl)-10-butyl-8,8,12,16-tetramethyl-4,17-dioxa-bicyclo[14.1.0]heptadecane-5,9-dione;
(1 S,3S,7S,1 10R,11S,12S,16R)-3-(2-Aminomethyl-benzoxazol-5-yl)-7,11-dihydroxy-10-butyl-8,8,12,16-tetramethyl-4,17-dioxa-bicyclo[14.1.0]heptadecane-5,9-dione;
(4S,7R,8S,9S,13Z,16S)-4,8-Dihydroxy-7-allyl-5,5,9,13-tetramethyl-16-(2-methyl-benzoxazol-5-yl)-oxacyclohexadec-13-ene-2,6-dione;
(4S,7R,8S,9S,13Z,16S)-4,8-Dihydroxy-16-(2-hydroxymethyl-benzoxazo1-5-yl)-7-allyl-5,5,9,13-tetramethyl-oxacyclohexadec-13-ene-2,6-dione;
(4S,7R,8S,9S,13Z,16S)-16-(2-Aminomethyl-benzoxazol-5-yl)-4,8-dihydroxy-7-allyl-5,5,9,13-tetramethyl-oxacyclohexadec-13-ene-2,6-dione;
(1S,3S,7S,10R,11S,12S,16R)-7,11-Dihydroxy-10-allyl-8,8,12,16-tetramethyl-3-(2-methyl-benzoxazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzoxazol-5-yl)-10-allyl-8,8,12,16-tetramethyl-4,17-dioxa-bicyclo[14.1.0]heptadecane-5,9-dione;
(1S,3S,7S,10R,11S,12S,16R)-3-(2-Aminomethyl-benzoxazol-5-yl)-7,11-dihydroxy-10-allyl-8,8,12,16-tetramethyl-4,17-dioxa-bicyclo[14.1.0]heptadecane-5,9-dione;
(4S,7R,8S,9S,13Z,16S)-4,8-Dihydroxy-7-prop-2-inyl-5,5,9,13-tetramethyl-16-(2-methyl-benzoxazol-5-yl)-oxacyclohexadec-13-ene-2,6-dione;
(4S,7R,8S,9S,13Z,16S)-4,8-Dihydroxy-16-(2-hydroxymethyl-benzoxazol-5-yl)-7-prop-2-inyl-5,5,9,13-tetramethyl-oxacyclohexadec-13-ene-2,6-dione;
(4S,7R,8S,9S,13Z,16S)-16-(2-Aminomethyl-benzoxazol-5-yl)-4,8-dihydroxy-7-prop-2-inyl-5,5,9,13-tetramethyl-oxacyclohexadec-13-ene-2,6-dione;
(1 S,3S,7S,10R,11S,12S,16R)-7,11-Dihydroxy-10-prop-2-inyl-8,8,12,16-tetramethyl-3-(2-methyl-benzoxazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzoxazol-5-yl)-10-prop-2-inyl-8,8,12,16-tetramethyl-4,17-dioxa-bicyclo[14.1.0]heptadecane-5,9-dione;
(1S,3S,7S,10R,11S,125,16R)-3-(2-Aminomethyl-benzoxazol-5-yl)-7,11-dihydroxy-10-prop-2-inyl-8,8,12,16-tetramethyl-4,17-dioxa-bicyclo[14.1.0]-heptadecane-5,9-dione;
(4S,7R,8S,9S,13Z,16S)-4,8-Dihydroxy-7-but-3-enyl-5,5,9,13-tetramethyl-16-(2-methyl-benzoxazol-5-yl)-oxacyclohexadec-13-ene-2,6-dione;
(4S,7R,8S,9S,13Z,16S)-4,8-Dihydroxy-16-(2-hydroxymethyl-benzoxazol-5-yl)-7-but-3-enyl-5,5,9,13-tetramethyl-oxacyclohexadec-13-ene-2,6-dione;
(4S,7R,8S,9S,13Z,16S)-16-(2-Aminomethyl-benzoxazol-5-yl)-4,8-dihydroxy-7-but-3-enyl-5,5,9,13-tetramethyl-oxacyclohexadec-13-ene-2,6-dione;
(1S,3S,7S,10R,11S,12S,16R)-7,11-Dihydroxy-10-but-3-enyl-8,8,12,16-tetramethyl-3-(2-methyl-benzoxazol-5-yl)-4,17-dioxa-bicyclo[14.1.0]heptadecane-5,9-dione;
(1S,3S,7S,10R, 11S,12S,16R)-7,11-Dihydroxy-3-(2-hydroxymethyl-benzoxazol-5-yl)-10-but-3-enyl-8,8,12,16-tetramethyl-4,17-dioxa-bicyclo[14.1.0]heptadecane-5,9-dione;
(1S,3S,7S,10R,11S,125,16R)-3-(2-Aminomethyl-benzoxazol-5-yl)-7,11-dihydroxy-10-but-3-enyl-8,8,12,16-tetramethyl-4,17-dioxa-bicyclo[14.1.0]heptadecane-5,9-dione;
(4S,7R,8S,9S,13Z,16S)-4,8-Dihydroxy-7-but-3-inyl-5,5,9,13-tetramethyl-16-(2-methyl-benzoxazol-5-yl)-oxacyclohexadec-13-ene-2,6-dione;
(4S,7R,8S,9S,13Z,16S)-4,8-Dihydroxy-16-(2-hydroxymethyl-benzoxazol-5-yl)-7-but-3-inyl-5,5,9,13-tetramethyl-oxacyclohexadec-13-ene-2,6-dione;
(4S,7R,8S,9S,13Z,16S)-16-(2-Aminomethyl-benzoxazol-5-yl)-4,8-dihydroxy-7-but-3-inyl-5,5,9,13-tetramethyl-oxacyclohexadec-13-ene-2,6-dione;
(1S,3S,7S,10R,11S,12S,16R)-7,11-Dihydroxy-10-but-3-inyl-8,8,12,16-tetramethyl-3-(2-methyl-benzoxazol-5-yl)-4,17-dioxa-bicyclo[14.1.0]heptadecane-5,9-dione;
(1S,3S,7S,10R,11S,12S,16R)-7,11-Dihydroxy-3-(2-hydroxymethyl-benzoxazol-5-yl)-10-but-3-inyl-8,8,12,16-tetramethyl-4,17-dioxa-bicyclo[14.1.0]heptadecane-5,9-dione;
(1S,3S,7S,10R,11S,12S,16R)-3-(2-Aminomethyl-benzoxazol-5-yl)-7,11-dihydroxy-10-but-3-inyl-8,8,12,16-tetramethyl-4,17-dioxa-bicyclo[14.1.0]heptadecane-5,9-dione.

In a compound of general formula (I) that contains one of the above-mentioned elements, the hydrogen atoms in the above-mentioned elements are replaced by radicals L¹-L³ in the positions indicated in formula (I), whereby radicals L¹-L³ have the above-indicated meanings.

The above-mentioned conjugates can comprise one or more recognition units; in this case, the recognition units that are related to a conjugate can be identical or different. It is preferred that the recognition units of a conjugate be identical.

The compounds of general formula I can be used in the form of their α-, β- or γ-cyclodextrin clathrates or their substituted α-, β- or γ-cyclodextrin clathrates or in the form of liposomal or PEGylated compositions.

In other preferred embodiments, the epothilone molecule contains a lactone or a lactame moiety.

Another preferred group of epothilones for use in the present invention are compounds of the general formula below: 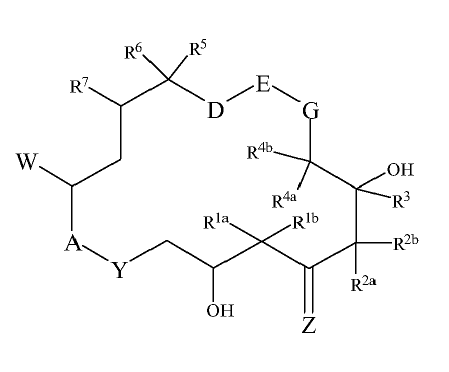 wherein:
- R^{1a}, R^{1b}: are each independently hydrogen, C₁-C₁₀ alkyl, aryl, aralkyl, or together form a -(CH₂)ₘ-group where m is 2 to 5;
- R^{2a}, R^{2b}: are each independently hydrogen, C₁-C₁₀ alkyl, aryl, aralkyl, or together form a -(CH₂)ₙ-group where n is 2 to 5, or are C₂-C₁₀ alkenyl or C₂-C₁₀ alkynyl;
- R³: is hydrogen, C₁-C₁₀ alkyl, aryl, aralkyl;
- R^{4a}, R^{4b}: are each independently hydrogen, C₁-C₁₀ alkyl, aryl, aralkyl, or together form a -(CH₂)ₚ- group where p is 2 to 5;
- R⁵: is hydrogen, C₁-C₁₀ alkyl, aryl, aralkyl, CO₂H CO₂alkyl, CH₂OH, CH₂Oalkyl, CH₂Oacyl, CN, CH₂NH₂, CH₂N(alkyl, acyl)_{1,2}, or CH₂Hal;
- R⁶, R⁷: are each hydrogen, or together form an additional bond, or together form an epoxy function;
- G: is O or CH₂;
- D-E: together is a group -H₂C-CH₂-, -HC=CH-, -C≡C-, -CH(OH)-CH(OH)-, -CH(OH)-CH₂-, -CH₂-CH(OH)-, -CH₂-O-, -O-CH₂- or 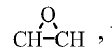 whereby if G is O then D-E cannot be CH₂-O; or
- D-E-G: together is a group H₂C-CH=CH
- W: is a group C(=X)R⁸, or is a bi- or tricyclic aromatic or heteroaromatic radical;
- X: is O, or two groups OR²⁰, or a C₂-C₁₀ alkylenedioxy group
(which may be straight or branched), or H and the group OR⁹, or a group CR¹⁰R¹¹;
- R⁸: is hydrogen, C₁-C₁₀ alkyl, aryl, aralkyl, halogen, CN;
- R⁹: is hydrogen or a protecting group PG^{x};
- R¹⁰, R¹¹: are each independently hydrogen, C₁-C₂₀ alkyl, aryl, aralkyl, or together with the methylene carbon form a 5- to 7-membered carbocyclic ring;
- Z: is O or H and the group OR¹²;
- R¹²: is hydrogen or a protecting group PG^{z};
- A-Y: is a group O-C(=O), O-CH₂, CH₂-C(=O), NR²¹-C(=O), NR²¹-SO₂;
- R²⁰: is a C₁-C₂₀ alkyl group;
- R²¹: is hydrogen, or C₁-C₁₀ alkyl;
- PG^{x}, PG^{z}: is C₁-C₂₀ alkyl, C₄-C₇ cycloalkyl, which may contain one or more oxygen atoms in the ring, aryl, aralkyl, C₁-C₂₀ acyl, aroyl, C₁-C₂₀ alkylsulfonyl, arylsulfonyl, tri(C₁-C₂₀ alkyl)silyl, di(C₁-C₂₀ alkyl) arylsilyl, (C₁-C₂₀ alkyl)diarylsilyl, or tri(aralkyl)silyl;
as a single stereoisomer or a mixture of different stereoisomers, and / or as a pharmaceutically acceptable salt thereof.

For all of the afore-mentioned epothilones and epothilone conjugates, the radicals shall be understood to have the following meanings:

As alkyl groups R^{1a}, R^{1b}, R^{2a}, R^{2b}, R³, R^{4a}, R^{4b}, R⁵, R⁸, R¹⁰, R¹¹, R²⁰, R²¹, R^{22a}, R^{22b}, R²³, R²⁵, R²⁶ and R²⁷, straight-chain or branched-chain alkyl groups with 1-20 carbon atoms can be considered, such as, for example, methyl, ethyl, propyl, isopropyl, butyl, isobutyl, tert.-butyl, pentyl, isopentyl, neopentyl, heptyl, hexyl, and decyl.

Alkyl groups R^{1a}, R^{1b}, R^{2a}, R^{2b}, R³, R^{4a}, R^{4b}, R⁵, R⁸, R¹⁰, R¹¹, R²⁰, R²¹, R^{22a}, R^{22b}, R²⁵, R²⁶ and R²⁷ can also be perfluorinated or substituted by 1-5 halogen atoms, hydroxy groups, C₁-C₄-alkoxy groups, or C₆-C₁₂-aryl groups (which can be substituted by 1-3 halogen atoms).

As aryl radicals R^{1a}, R^{1b}, R^{2a}, R^{2b}, R³, R^{4a}, R^{4b}, R⁵, R⁸, R¹⁰, R¹¹, R^{22a}, R^{22b}, R²⁶ and R²⁷, substituted and unsubstituted carbocyclic or heterocyclic radicals with one or more heteroatoms, such as phenyl, naphthyl, furyl, thienyl, pyridyl, pyrazolyl, pyrimidinyl, oxazolyl, pyridazinyl, pyrazinyl, quinolyl, thiazolyl, benzothiazolyl, benzoxazolyl, which can be substituted in one or more places by halogen, OH, O-alkyl, CO₂H, CO₂-alkyl, -NH₂, -NO₂, -N₃, -CN, C₁-C₂₀-alkyl, C₁-C₂₀-acyl, C₁-C₂₀-acyloxy groups, are suitable. The heteroatoms can be oxidized if as a result the aromatic character is not lost, such as, for example, the oxidation of a pyridyl to a pyridyl-N-oxide.

As bi- and tricyclic aryl radicals W, substituted and unsubstituted carbocyclic or heterocyclic radicals with one or more heteroatoms, such as naphthyl, anthryl, benzothiazolyl, benzoxazolyl, benzimidazolyl, quinolyl, isoquinolyl, benzoxazinyl, benzofuranyl, indolyl, indazolyl, quinoxalinyl, tetrahydroisoquinolinyl, tetrahydroquinolinyl, thienopyridinyl, pyridopyridinyl, benzopyrazolyl, benzotriazolyl, or dihydroindolyl, which can be substituted in one or more places by halogen, OH, O-alkyl, CO₂H, CO₂-alkyl, -NH₂, -NO₂, -N₃, -CN, C₁-C₂₀-alkyl, C₁-C₂₀-acyl, or C₁-C₂₀-acyloxy groups, are suitable. The heteroatoms can be oxidized if as a result the aromatic character is not lost, such as, for example, the oxidation of a quinolyl to a quinolyl-N-oxide.

The aralkyl groups in R^{1a}, R^{1b}, R^{2a}, R^{2b}, R³, R^{4a}, R^{4b}, R⁵, R⁸, R¹⁰, R¹¹, R^{22a}, R^{22b}, R²⁶ and R²⁷ can contain in the ring up to 14 C atoms, preferably 6 to 10 C atoms, and in the alkyl chain 1 to 8, preferably 1 to 4 atoms. As aralkyl radicals, for example, benzyl, phenylethyl, naphthylmethyl, naphthylethyl, furylmethyl, thienylethyl, and pyridylpropyl are considered. The rings can be substituted in one or more places by halogen, OH, O-alkyl, CO₂H, CO₂-alkyl, -NO₂, -N₃, -CN, C₁-C₂₀-alkyl, C₁-C₂₀-acyl, or C₁-C₂₀-acyloxy groups.

As representatives of protective groups PG, tris(C₁-C₂₀ alkyl)silyl, bis(C₁-C₂₀ alkyl)-arylsilyl, (C₁-C₂₀ alkyl)-diarylsilyl, tris(aralkyl)-silyl, C₁-C₂₀-alkyl, C₂-C₂₀-alkenyl, C₄-C₇-cycloalkyl, which in addition can contain an oxygen atom in the ring, aryl, C₇-C₂₀-aralkyl, C₁-C₂₀-acyl, aroyl, C₁-C₂₀-alkoxycarbonyl, C₁-C₂₀-alkylsulfonyl as well as arylsulfonyl can be mentioned.

As alkyl, silyl and acyl radicals for protective groups PG, in particular the radicals that are known to one skilled in the art are considered. Preferred are the alkyl or silyl radicals that are easily cleavable from the corresponding alkyl ethers and silyl ethers, such as, for example, the methoxymethyl, methoxyethyl, ethoxyethyl, tetrahydropyranyl, tetrahydrofuranyl, trimethylsilyl, triethylsilyl, tert.-butyldimethylsilyl, tert.-butyldiphenylsilyl, tribenzylsilyl, triisopropylsilyl, benzyl, para-nitrobenzyl, or para-methoxybenzyl radical as well as alkylsulfonyl and arylsulfonyl radicals. As an alkoxycarbonyl radical, e.g., trichloroethyloxycarbonyl (Troc) is suitable. As acyl radicals, e.g., formyl, acetyl, propionyl, isopropionyl, trichloromethylcarbonyl, pivalyl, butyryl or benzoyl, which can be substituted with amino groups and/or hydroxy groups, are suitable.

As amino protective groups PG, the radicals that are known to one skilled in the art are considered. For example, the Alloc, Boc, Z, benzyl, f-Moc, Troc, Stabase or benzostabase group can be mentioned.

As halogen atoms, fluorine, chlorine, bromine or iodine is considered.

The acyl groups can contain 1 to 20 carbon atoms, whereby formyl, acetyl, propionyl, isopropionyl and pivalyl groups are preferred.

The term N(alkyl,acyl)_{1,2} includes N(alkyl)₂, N(alkyl)(acyl), N(acyl)₂ whereby the alkyl group is C₁-C₅-alkyl as defined above, and the acyl group is defined as above but includes also benzoyl.

The C₂-C₁₀-alkylene-α,ω-dioxy group that is possible for X is preferably an ethyleneketal or neopentylketal group.

As preferred recognition units EG, those are considered that, for example, by overexpression of suitable enzymes in proliferating tissues can be cleaved from the latter. For example, glucuronidase can be mentioned in this regard.

Preferred compounds of general formula I are those in which A-Y represents O-C(=O) or NR²¹-C(=O); D-E represents an H₂C-CH₂ group or an HC=CH group; G represents a CH₂ group; Z represents an oxygen atom; R^{1a}, R^{1b} in each case represent C₁-C₁₀ alkyl or together a -(CH₂)ₚ group with p equal to 2 or 3 or 4; R^{2a}, R^{2b}, independently of one another, represent hydrogen, C₁-C₁₀ alkyl, C₂-C₁₀ alkenyl, or C₂-C₁₀ alkinyl; R³ represents hydrogen; R^{4a}, R^{4b}, independently of one another, represent hydrogen or C₁-C₁₀ alkyl; R⁵ represents hydrogen or C₁-C₄ alkyl or CH₂OH or CH₂NH₂ or CH₂N(alkyl, acyl)_{1,2} or CH₂Hal; R⁶ and R⁷ together represent an additional bond or together an oxygen atom or together an NH group or together an N-alkyl group or together a CH₂ group; W represents a group C(=X)R⁸ or a 2-methylbenzothiazol-5-yl radical or a 2-methylbenzoxazol-5-yl radical or a quinolin-7-yl radical or a 2-aminomethylbenzothiazol-5-yl radical or a 2-hydroxymethylbenzothiazol-5-yl radical or a 2-aminomethylbenzoxazol-5-yl radical or a 2-hydroxymethyl-benzoxazol-5-yl radical; X represents a CR¹⁰R¹¹ group; R⁸ represents hydrogen or C₁-C₄ alkyl or a fluorine atom or a chlorine atom or a bromine atom; R¹⁰/R¹¹ represent hydrogen/2-methylthiazol-4-yl or hydrogen/2-pyridyl or hydrogen/2-methyloxazol-4-yl or hydrogen/2-aminomethylthiazol-4-yl or hydrogen/2-aminomethyloxazol-4-yl or hydrogen/2-hydroxymethylthiazol-4-yl or hydrogen/2-hydroxymethyloxazol-4-yl.

In a preferred embodiment, radicals R^{22a} and R^{22b} are selected from the group that consists of C₁-C₈-alkyl, C₁-C₈-alkoxy, halogen, nitro, CN, N₃, NH₂ and CO₂-(C₁-C₈-alkyl). Especially preferred in this connection are the radicals methyl, ethyl, propyl, i-propyl, t-butyl, CF₃, C₂F₅, F, Cl, nitro, CN, N₃, NH₂, CO₂-methyl, CO₂-ethyl, CO₂-propyl and CO₂-i-propyl.

In another preferred embodiment, radical R²⁶ is selected from the group that consists of C₁-C₈-alkyl and C₂-C₈-alkenyl. Especially preferred in this connection are the radicals methyl, ethyl, propyl, i-propyl, t-butyl, CF₃, propenyl and butenyl.

In another preferred embodiment, radicals R^{2a} and R^{2b} are selected such that one of radicals R^{2a} or R^{2b} represents hydrogen, while the other radical in each case is selected from the group that consists of C₁-C₇-alkyl, C₂-C₇-alkenyl and C₂-C₇-alkinyl. Especially preferred in this connection are the radicals methyl, ethyl, propyl, i-propyl, propenyl, butenyl, propinyl and butinyl.

Preferred linker-recognition units have general formula III¹: 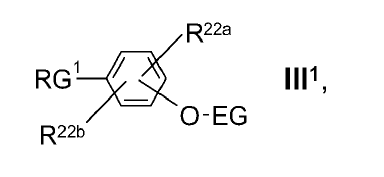 in which
- RG¹: represents an O=C=N group or an S=C=N group or an
O=C=N-CH₂ group or an S=C=N-CH₂ group; and
R^{22a}, R^{22b} and EG have the above-indicated meanings;
as well as linker-recognition units of general formula III²: 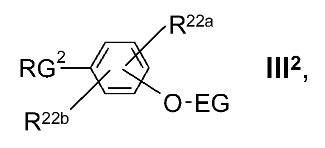 in which
- RG²: represents an HO-CH₂ group or an HNR²³-CH₂ group; and
R^{22a}, R^{22b} and EG have the above-mentioned meanings;
but with the condition that the following compounds are not included:
(4-Hydroxymethyl)phenyl-2,3,4,6-tetra-O-acetyl-α-D-galactopyranoside;
(2-Hydroxymethyl)phenyl-2,3,4,6-tetra-O-acetyl-α-D-galactopyranoside;
(4-Hydroxymethyl)phenyl-2,3,4-tri-O-acetyl-α-D-glucuronide-6-methyl ester;
(2-Hydroxymethyl)phenyl-2,3,4-tri-O-acetyl-α-D-glucuronide-6-methyl ester;
(4-Hydroxymethyl)phenyl-2,3,4,6-tetra-O-acetyl-α-D-glucopyranoside;
(2-Hydroxymethyl-4-nitro)phenyl-2,3,4,6-tetra-O-acetyl-α-D-galactopyranoside;
(4-Hydroxymethyl-2-nitro)phenyl-2,3,4,6-tetra-O-acetyl-α-D-galactopyranoside;
(2-Hydroxymethyl-4-nitro)phenyl-2,3,4-tri-O-acetyl-α-D-glucuronide-6-methyl ester;
(4-Hydroxymethyl-2-nitro)phenyl-2,3,4-tri-O-acetyl-α-D-glucuronide-6-methyl ester;
(2-Chloro-4-hydroxymethyl)phenyl-2,3,4,6-tetra-O-acetyl-α-D-galactopyranoside;
(2-Chloro-4-hydroxymethyl)phenyl-2,3,4-tri-O-acetyl-β-D-glucuronide-6-methyl ester; as well as linker-recognition units of general formula III³: 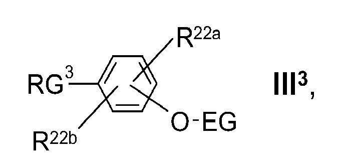 in which
- RG³: represents a Hal-C(=O)-CH₂ group or a Hal-C(=S)-CH₂ group or an
R²⁷-C(=O)-O-C(=O)-CH₂ group or an R²⁷-C(=O)-O-C(=S)-CH₂ group or a 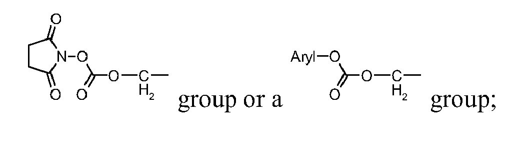
- R²⁷: is C₁-C₁₀ alkyl, aryl or aralkyl; and
R^{22a}, R^{22b} and EG have the above-mentioned meanings;
but with the condition that the following compounds are not included:
2,5-Dioxopyrrolidin-1-yl-[4-(2,3,4,6-tetra-O-acetyl-α-D-galactopyranosyl)-benzyl] carbonate;
2,5-Dioxopyrrolidin-1-yl-[2-(2,3,4,6-tetra-O-acetyl-α-D-galactopyranosyl)-benzyl] carbonate;
2,5-Dioxopyrrolidin-1-yl-[4-((2,3,4-tri-O-acetyl-β-D-glucopyranosyl)-methyluronate)benzyl]carbonate;
4-Nitrophenyl-[2-((2,3,4-tri-O-acetyl-β-D-glucopyranosyl)methyluronate)-benzyl] carbonate;
2,5 -Dioxopyrrolidin-1 -yl-[4-(2,3,4,6-tetra-O-acetyl-β-D-glucopyranosyl)benzyl]-carbonate;
4-Nitrophenyl-[2-(2,3,4,6-tetra-O-acetyl-α-D-galactopyranosyl)-5-nitrobenzyl]-carbonate;
4-Nitrophenyl-[2-((2,3,4-tri-O-acetyl-β-D-glucopyranosyl)methyluronate)-5-nitrobenzyl]carbonate;
4-Nitrophenyl-[4-methoxy-5-nitro-2-((2,3,4-tri-O-acetyl-β-D-glucopyranosyl)methyluronate)benzyl] carbonate;
4-Nitrophenyl-[4-((2,3,4-tri-O-acetyl-β-D-glucopyranosyl)methyluronate)-5-nitrobenzyl]carbonate;
4-Chlorophenyl-[2-((2,3,4-tri-O-acetyl-β-D-glucopyranosyl)methyluronate)-5-nitrobenzyl]carbonate.

In certain preferred embodiments, the epothilone conjugates may contain more than one recognition unit, optionally wherein the recognition units are identical. The recognition unit may, for example, be selected from an antibody, or an antigen-binding fragment of the same which is specific to an antigen that is selected from the group consisting of CD19, CD20, CD40, CD22, CD25, CD5, CD52, CD10, CD2, CD7, CD33, CD38, CD40, CD72, CD4, CD21, CD37, CD30, VCAM, CD31, ELAM, endoglin, VEGFRI/II, VEGFRvIII, scFv(14E1), αvβ3, Tie1/2, TES23 (CD44ex6), phosphatidylserine, PSMA, VEGFR/VEGF complex and ED-B-fibronectin.

Particularly preferred epothilones or epothilone analogs or derivative in the context of the present invention include epothilone A, epothilone B, epothilone C, 13-alkyl-epothilone C derivatives, epothilone D, trans-epothilone D, epothilone E, epothilone F, an effector conjugate of epothilone, ZK-EPO (sagopilone), or any of the epothilones referred to in the literature as ixabepilone (BMS-247550), BMS-310705, EPO-906, Patupilone, Kos-862, Kos-1584, Kos-1803, ABJ 879. Of course, any pharmaceutically acceptable salt of the aforementioned epothilones shall also be included herein.

The following examples have been performed with a preferred epothilone derivative named ZK-EPO or sagopilone which is (1S,3S,7S,10R,11S,12S,16R)-7,11-Dihydroxy-10-allyl-8,8,12,16-tetramethyl-3-(2-methyl-benzothiazol-5-yl)-4,17-dioxa-bicyclo[14.1.0]heptadecane-5,9-dione.

The production of epothilones, their precursors and derivatives is generally carried out according to the methods known to one skilled in the art. Suitable methods are, for example, described in DE 19907588, WO 98/25929, WO 99/58534, WO 99/2514, WO 99/67252, WO 99/67253, WO 99/7692, EP 99/4915, WO 00/485, WO 00/1333, WO 00/66589, WO 00/49019, WO 00/49020, WO 00/49021, WO 00/71521, WO 00/37473, WO 00/57874, WO 01/92255, WO 01/81342, WO 01/73103, WO 01/64650, WO 01/70716, US 6204388, US 6387927, US 6380394, US 02/52028, US 02/58286, US 02/62030, WO 02/32844, WO 02/30356, WO 02/32844, WO 02/14323, and WO 02/8440.

It will be apparent to those of skill in the art that many modifications and variations of the embodiments described herein are possible without departing from the spirit and scope of the present invention. The present invention and its advantages are further illustrated in the following, non-limiting examples.

### Examples

### Example 1: Lung cancer xenograft models

Lung cancer xenograft models were established by subcutaneously transplanting fresh tumor material from NSCLC patients into nude mice. Tumor types included pleomorphic carcinoma (7187, 7336), squamous cell carcinoma (7126, 7177, 7298, 7343, 7462, 7433, 7530), dedifferentiated carcinoma (7668), adenocarcinoma (7064, 7198, 7406, 7387, 7466, 7612) and small cell lung cancer (7530). A schematic overview of the study design is presented in Figure 1.

It will be appreciated that the in vivo model is not limited to the analysis of the above-mentioned carcinoma, but can also be used to analyze the reaction of a patient to a compound or mixture of compounds having another type of cancer or disease.

Antitumor activity was determined using either the reduction in tumor area in treated versus control mice (T/C ratio), or the mean change in absolute tumor volume compared with volume at baseline (day of first treatment).

1° lung cancer biopsies obtained from patients with established cancer were transplanted into nude mice. Tumor growth curves for the treatment with Etoposide, Carboplatin, Gemcitabine, Taxol, Navelbine, Cetuximab, Erlotinib und ZK-EPO (sagopilone) were generated. Mutation status of p53 was analyzed in the models according to methods known in the art. Seven ZK-EPO responders and four ZK-EPO non-responders were characterized in long-term experiments. A PBS control group was treated with PBS or ZK-EPO for 24 hrs before sacrifice. RNA for ZK-EPO responder and non-responder models were isolated according to methods known in the art and were hybridized onto arrays according to the manufacturer's instructions.

As demonstrated herein, ZK-EPO showed superior tumor growth inhibition activity across the range of NSCLC xenografts. ZK-EPO exhibits superior activity and tolerability compared with commonly used cytotoxic agents in NSCLC, such as carboplatin, paclitaxel, gemcitabine, etoposide and vinorelbine.

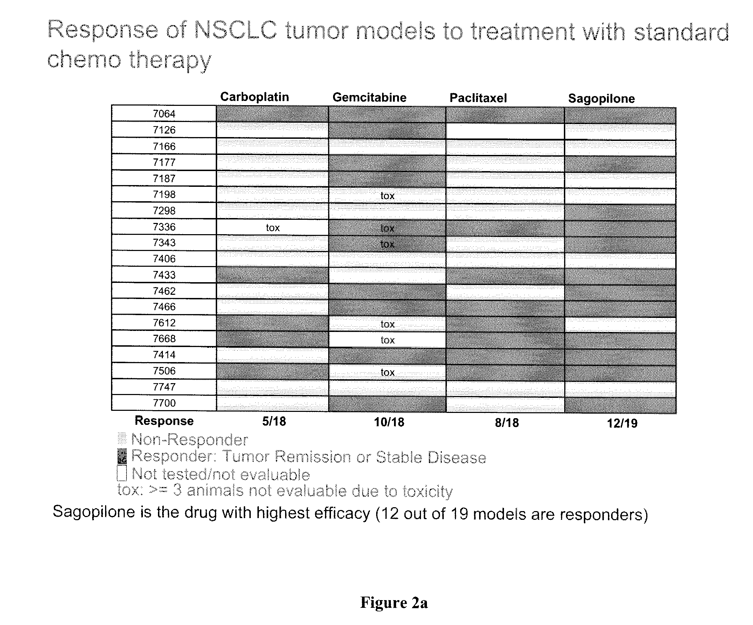

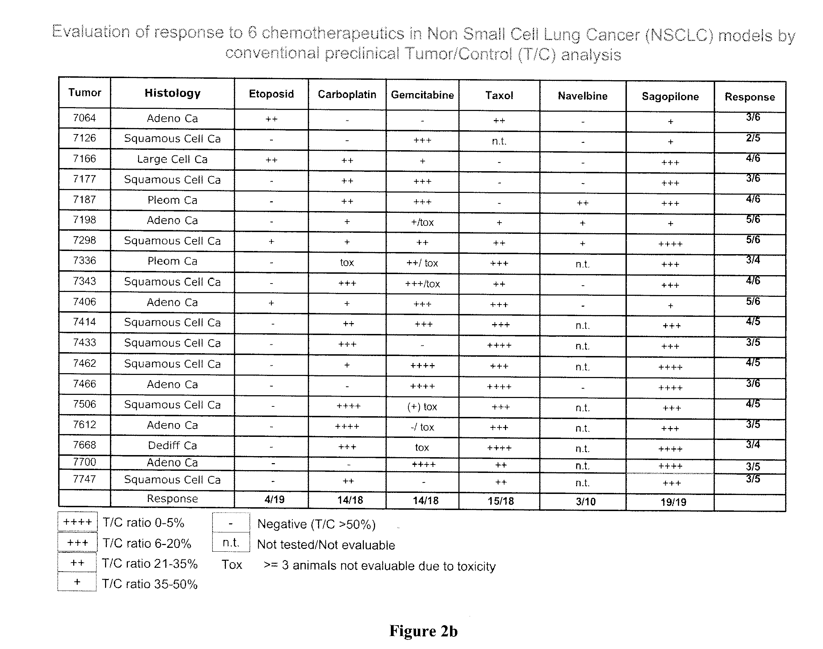

When the sensitivity of 17 different lung cancer xenografts to ZK-EPO and other antitumor treatments was analyzed (Figures 2a and 2b), ZK-EPO was the only agent that inhibited tumor growth in all 17 lung tumors, and remarkably in 12 NSCLC (76.5%) with a T/C ratio <20% which means a growth reduction of more than 80%. The activity of ZK-EPO was compared with standard chemotherapies used in the management of NSCLC (carboplatin, paclitaxel, gemcitabine, etoposide and vinorelbine).

Of the other chemotherapeutic drugs evaluated, gemcitabine also resulted in a high percentage of responses (80.0% overall and 47.0% with a T/C ratio <20%) but was associated with marked toxicity, unlike ZK-EPO. Paclitaxel elicited a response in 81.2% of tumors with 56.2% having a T/C ratio <20% and carboplatin resulted in a response in 68.8% of tumors but with a T/C ratio <20% in only 25.0% of the samples.

Although the response with gemcitabine was also high (61.5%), it should be noted that, in contrast to ZK-EPO, at least 2 animals per group (n=6) could not be evaluated in seven of the models due to toxicity, and toxicity prevented evaluation of any mice in 2 of the models.

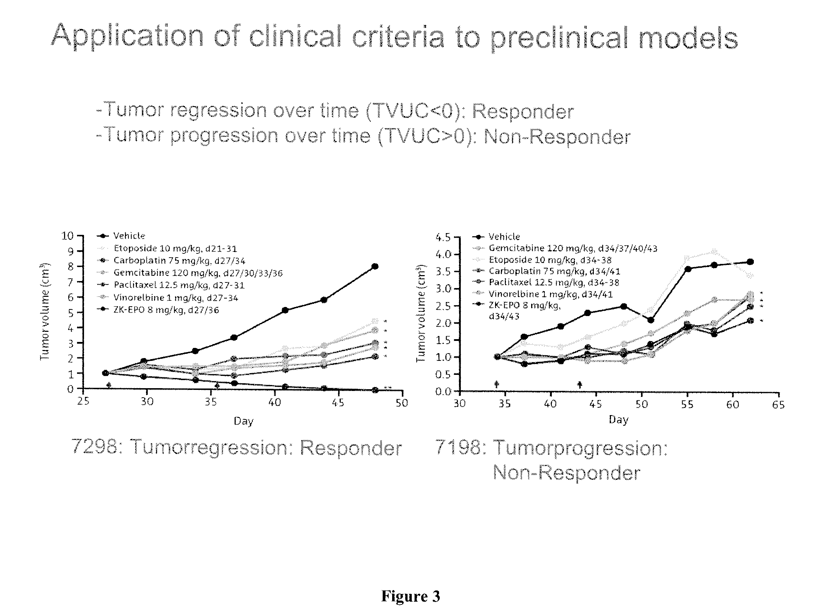

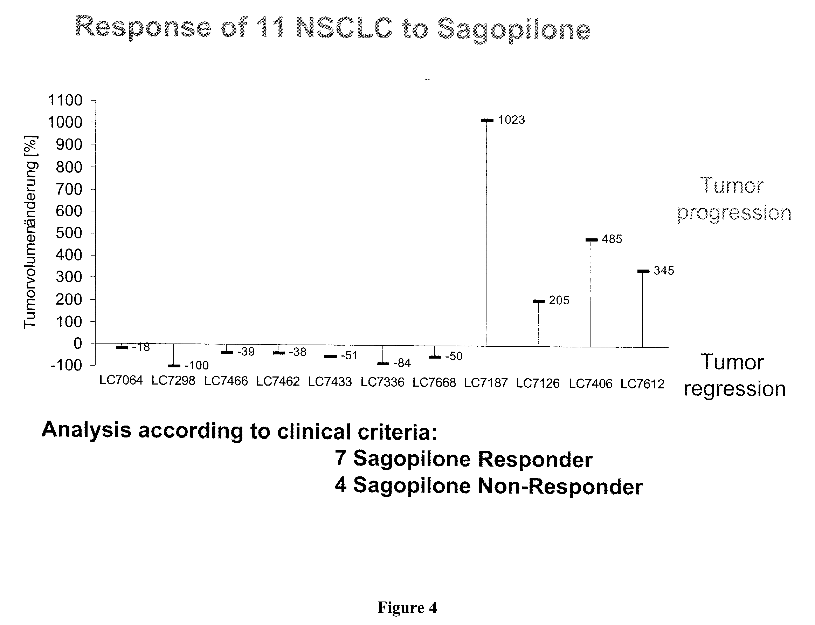

In summary, ZK-EPO exhibited a superior reduction in tumor volume relative to control (vehicle) compared with other agents commonly used in NSCLC treatment, including carboplatin, paclitaxel, gemcitabine, etoposide and vinorelbine (Figure 3). ZK-EPO is highly active against a series of new NSCLC xenograft models. Response of the lung xenograft models is highest with ZK-EPO. Figure 4 shows the response of 11 NSCLC xenograft models to sagopilone. Seven samples showed a tumor regression (responder) and four showed a tumor progression, (non-responder) after treatment with sagopilone. The seven ZK-EPO responders and four ZK-EPO non-responders were further characterized in long-term tumor growth experiments.

### Example 2: Analysis of expressed genes

2 x 2 x 2 mm³ tumor tissue samples were taken from the sacrificed animals. Samples were snap frozen and stored in liquid nitrogen until use. Total RNA of tumor samples was prepared using the RNeasy^{®} Mini Kit (Qiagen, Hilden, Germany) according to the manufacturer's recommendations. A DNase I (Qiagen) digestion step was included to eliminate genomic DNA. The quality of the total RNA was checked for integrity using the RNA LabChips on the Agilent Bioanalyzer 2100 (Agilent Technologies Inc., USA) and for and concentration on the Peqlab NanoDrop.

Affymetrix gene expression profiling (Affymetrix Inc., California, USA) was performed on primary tumors and passaged xenografts in accordance with the manufacturer's instructions.

The One-Cycle eukaryotic target labeling assay from Affymetrix (PN 900493) was used according to manufacturer's instructions. Briefly, 2 µg of high quality total RNA was reverse transcribed using T7 tagged oligo-dT primer in the first-strand cDNA synthesis reaction. After RNase H-mediated second-strand cDNA synthesis, the doublestranded cDNA was purified and served as template for the subsequent in vitro transcription reaction which generates biotin-labeled complementary RNA (cRNA). The biotinylated cRNA are then cleaned up, fragmented and hybridized to GeneChip HGU133Plus2.0 expression arrays (Affymetrix, Inc., Santa Clara, CA, U.S.A.), which contain about 54675 probe sets. The GeneChips were washed, and stained with streptavidin-phycoerythrin on a GeneChip Fluidics Station 450 (Affymetrix, Inc., Santa Clara, CA, U.S.A.). After washing on a GeneChip Fluidics Station 450, the arrays were scanned on an Affymetrix GeneChip 3000 scanner with autoloader and barcode reader (Affymetrix, Inc., Santa Clara, CA, U.S.A.). HGU133Plus2.0 arrays were used in accordance with the manufacturer's instructions.

### Example 3: Identification of differentially expressed genes

Objectives of the Affymetrix HGU133Plus2.0 study were the identification of genes which are differentially regulated by ZK-EPO and/or paclitaxel in concentrations representing the aneuploid (2.5 / 4 nM) and mitotic-arrest (40 nM) population of A549 cells - potentially indicating response markers.

The quality of the hybridized arrays was analyzed with the Expressionist Pro 4.0 Refiner (GeneData, Basel, CH) software. Here, based on raw intensities of individual oligonucleotide features (probes), the following analyses are performed: the experiments are grouped according to similarity and potential outlier experiments can be removed (or selected for re-hybridization, re-fragmentation), the quality of a particular experiment is compared with a virtual reference experiment, which is computed as average of all feature intensities of all arrays in that group. Moreover, defects on the arrays are masked. Here, for each array, the spatial signal distribution is compared with the reference experiment of the experiment group it belongs to. Regions with sharp boundaries, which have consistently higher or lower feature intensities compared to the reference experiment are flagged as defects and excluded from further analysis. In addition, a signal correction (distortion and gradient) is performed, the control gene statistics are calculated, and an overall classification of the quality of the experiments is provided.

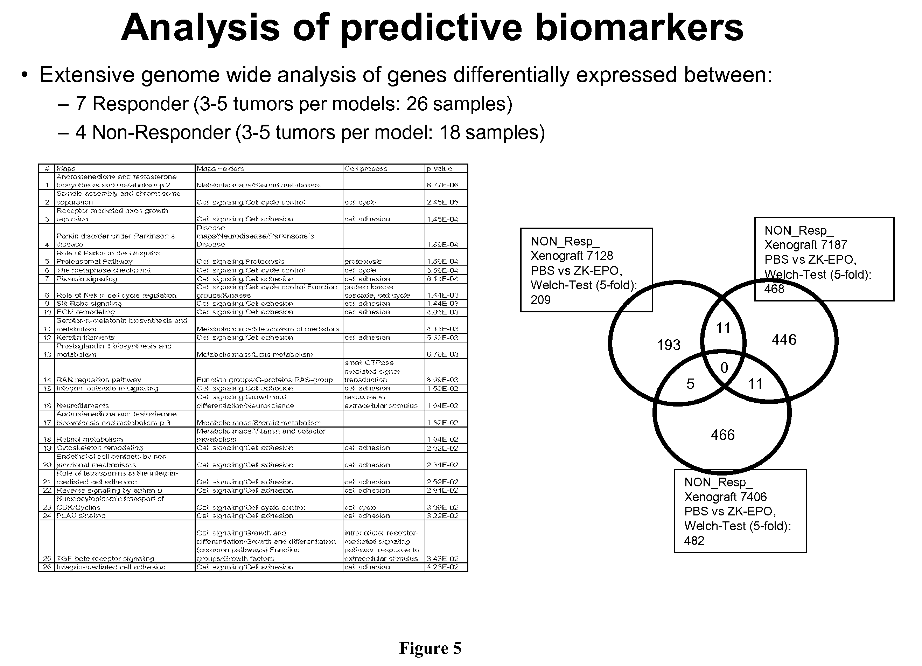

The probe intensities on each array were summarized with the MAS5.0 summarization algorithm and the refined and summarized data were loaded into the CoBi database (Genedata, Basel, CH). The analysis of the probe set specific signal intensities was performed with the Expressionist Pro 4.0 Analyst (GeneData, Basel, CH) software. The Analysis using Expressionist Analyst comprises Box plot, Box plot after median normalization, Global Analyses, Principal component analysis, Hierarchical clustering, Marker gene analysis: regulated gene sets from A549/ZK-EPO study, Valid value proportion and Statistical tests: t-test with Welch correction. The data set was median normalized (see Figure 5).

### Example 4: Analysis of potential biomarkers for epothilone responsiveness

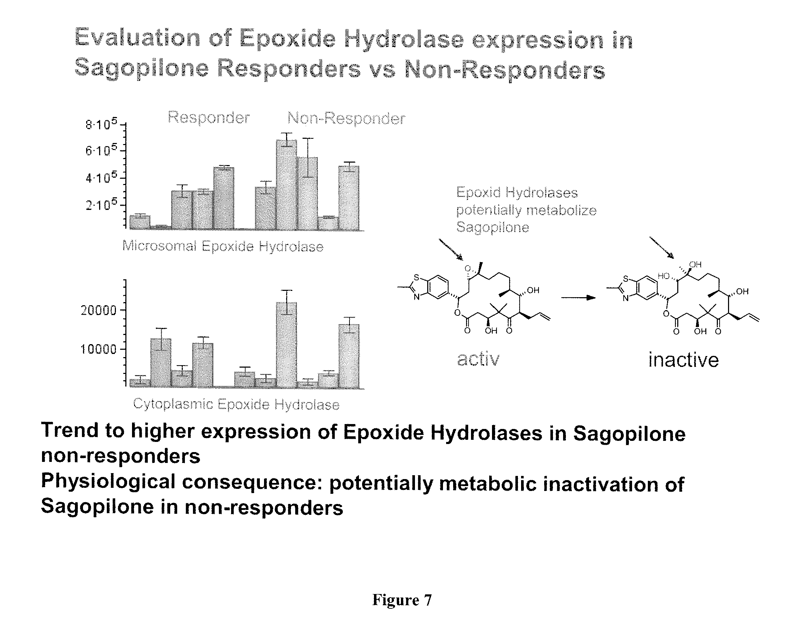

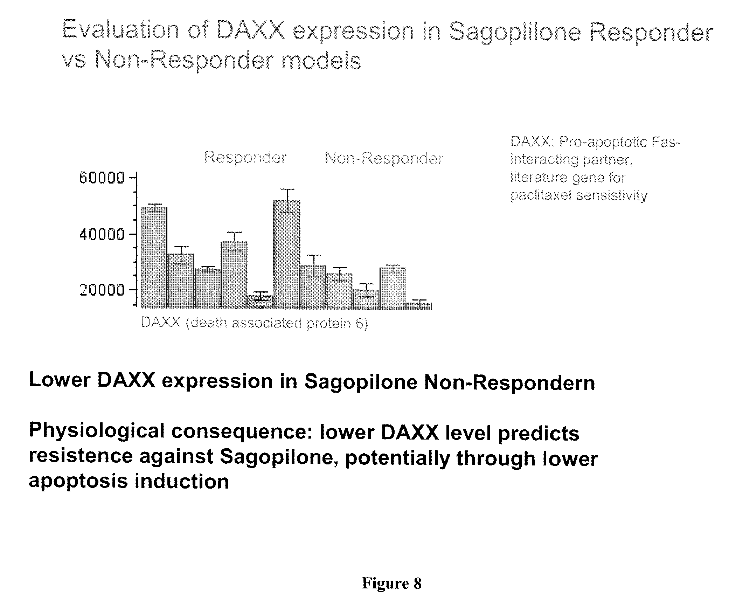

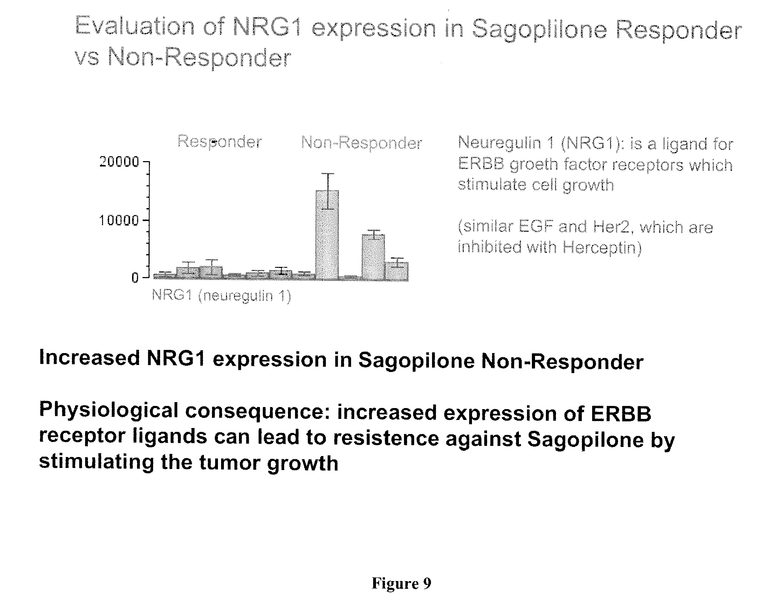

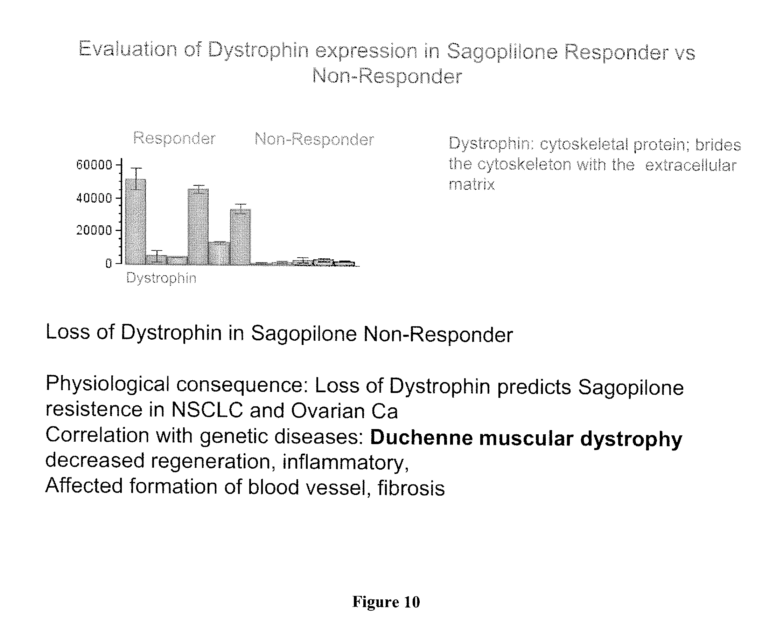

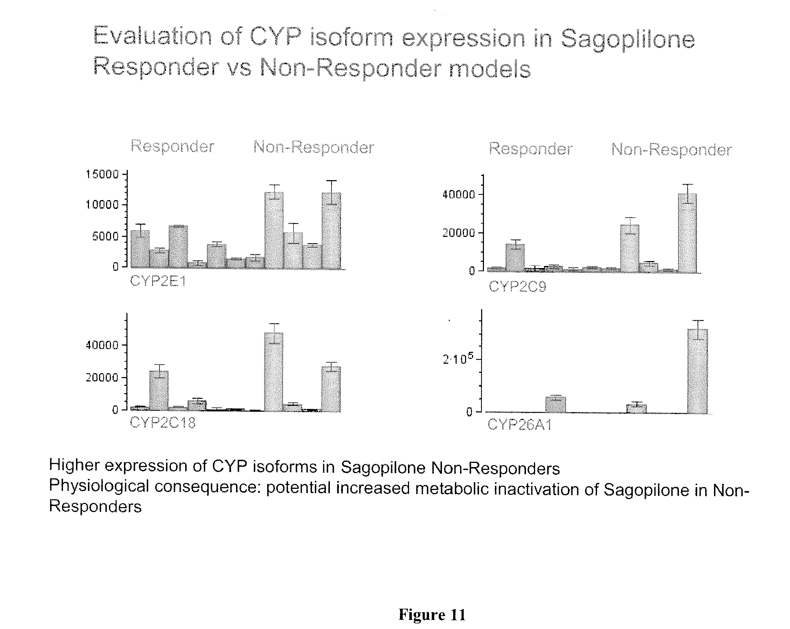

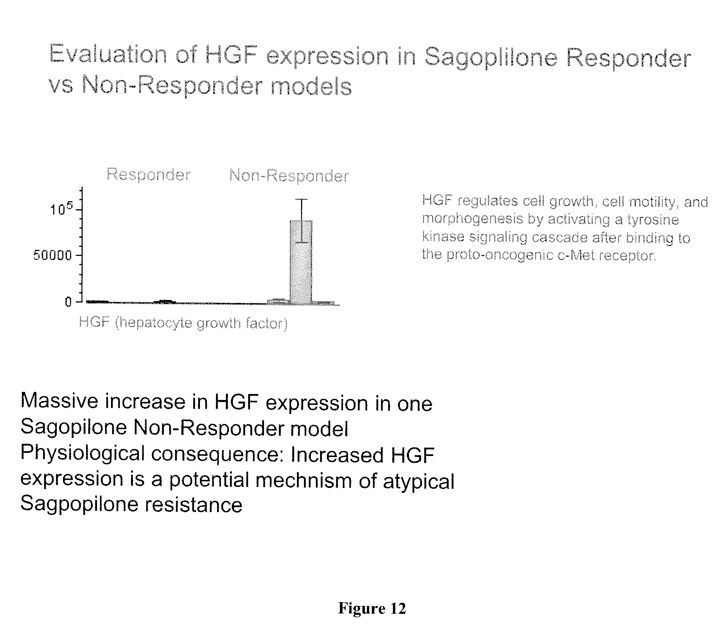

An analysis of potential biomarkers was carried out to compare the gene expression pattern of responders to sagopilone vs non-responders using the methods disclosed in Example 2. The Welch-test showed a good equivalence between the non-responders to other non-responders and remarkable differences between non-responders to responders. Closer analysis of the p53 status was performed as shown in Figure 6. Furthermore, the expression level of epoxide hydrolase, DAXX (pro-apoptotic Fas-interacting partner), NRG1 (Neuregulin 1), dystrophin, CYP (cytochrome P isoforms) and HGF (hepatocyte growth factor) were analyzed (see Figures 7-12).

The presence of a mutated p53 gene leading to a loss of function of the p53 protein is indicative of a potential responsiveness of said subject to an epothilone such as sagopilone. The increased gene expression of DAXX and dystrophin, indicate a potential responsiveness of said subject to an epothilone such as sagopilone. Increased gene expression of a CYP isoform, microsomal epoxide hydrolase, cytoplasmic epoxide hydrolase, NRG1, and/or HGF is indicative of a potentially decreased responsiveness of said subject to treatment with sagopilone.

Accordingly, a set of candidate genes/pathways has been identified which differentiate epothilone responders from non-responders. The down- or up-regulation of several gene expressions or protein levels, e.g. VEGF, GLUT1, aldolaseA, heme oxygenase, NIP3, PGK1, transferrin and their effects to pathways in which the genes and proteins are involved are shown in Figures 13 and 14.

### Example 5: Comparison of xenograft models and primary tumors

Representative samples of primary tumors and passaged xenografts were processed for histology, and sections were stained to evaluate tissue histology (H&E staining) and to determine the expression level of several proteins.

It was found that NSCLC tumor xenografts are indeed comparable to primary tumors. After first passage in nude mice, the histology and cell surface protein expression of the xenografts compared well to that of primary tumors, indicating that the xenografts retained their primary tumor characteristics. Gene expression profiling demonstrated that the xenografted tumors cluster together with the corresponding primary tumor after various passages. In addition, a comparison showed a good correlation between the various samples. This profiling demonstrates that after xenotransplantation in nude mice, the grown tumors had not significantly altered their characteristics, compared with their corresponding patient tumor. The models described in the present invention represent a valuable tool for the profiling of prospective anticancer compounds.

## Claims

1. A method for determining potential responsiveness of a subject to treatment of a pathologic disorder with an epothilone, wherein said method comprises analyzing in a sample from said subject the expression level of at least one gene or the protein encoded by it, wherein the protein is selected from the group consisting of a cytochrome P (CYP) isoform, microsomal epoxide hydrolase, cytoplasmic epoxide hydrolase, p53, pro-apoptotic Fas-interacting partner (DAXX), neuregulin 1 (NRG1), hepatocyte growth factor (HGF), dystrophin, UGT1A1, UGT1A3, UGT1A4, UGT1A8, UGT1A10, AKR1C2, VEGF, GLUT1, aldolaseA, heme oxygenase, NIP3, PGK1, transferrin, and HIF-prolyl hydroxylase.

2. The method of claim 1, wherein the presence of a mutated p53 gene leading to a loss of function, or wherein increased gene expression of DAXX and/or dystrophin is indicative of a potential responsiveness of said subject to said epothilone *(epothilone responder).*

3. The method of claim 1, wherein increased gene expression of a CYP isoform, microsomal epoxide hydrolase, cytoplasmic epoxide hydrolase, NRG1, and/or HGF is indicative of a potentially decreased responsiveness of said subject to treatment with said epothilone *(epothilone non-responder).*

4. A method for determining potential responsiveness of a subject to treatment of a pathologic disorder with an epothilone, wherein said method comprises analyzing the expression levels of multiple genes in a sample from said subject; and detecting the presence of a pathway deregulation by comparing the expression levels of the genes to a reference profile indicative of pathway deregulation, wherein the presence of pathway deregulation is indicative of a potentially decreased responsiveness of said subject to treatment with said epothilone *(epothilone non-responder).*

5. The method of claim 4, wherein the pathway that is deregulated is selected from the group consisting of the UDP glucuronosyl-transferases pathway and the response to hypoxia (HIF1A) pathway.

6. The method of claim 5, wherein the deregulation of the UDP glucuronosyl-transferases pathway is **characterized by** an increased expression of at least one gene coding for a protein selected from the group consisting of UGT1A1, UGT1A3, UGT1A4, UGT1A8, UGT1A10, and AKR1C2, or wherein the deregulation of the Response to Hypoxia (HIF1A) pathway is **characterized by** an increased expression of at least one gene coding for a protein selected from the group consisting of VEGF, GLUT1, aldolaseA, heme oxygenase, NIP3, PGK1, transferrin, and HIF-prolyl hydroxylase.

7. The method of any one of claims 1 to 6, wherein the determination of the expression profile is carried out *ex vivo* or *in vitro.*

8. The method of any one of claims 2, 3, 5, 6, and 7, wherein the expression level of said marker protein is increased by a factor of at least about 1.5, preferably at least about 2, alternatively wherein the expression level of said marker protein is increased by a factor of at least about 3, and preferably at least about 5.

9. The method of any one of claims 1 to 8, wherein the gene expression profile is obtained by the use of a microarray, preferably by the use of a Protein-, RNA- or cDNA-based microarray, or an SNP array.

10. The method of any one of claims 1 to 8, wherein the gene expression profile is determined quantitatively by a PCR-based technology, preferably wherein the PCR based technology is quantitative RT-PCR, alternatively wherein the gene expression profile is determined by fluorescence in situ hybridization (FISH) techniques.

11. The method of any one of claims 1 to 8, wherein the protein expression for each protein corresponding to the gene expression profile is determined by using western blot technology, ELISA, RIA, immunohistochemistry methods, mass spectrometry technologies, or antibody-based technologies, including protein microarrays and tissue arrays.

12. The method of claim 11, wherein the antibody further comprises a detectable label, preferably wherein the label is a radioactive label or a fluorescence label.

13. A method for determining a marker for potential responsiveness of a subject suffering from a malignant disorder to the treatment of said disorder with an epothilone, wherein said method comprises transferring tumor cells from a subject into nude mice, determining whether the primary tumor, or optionally a passaged xenograft tumor on mice or rats or tissue from these animals, is responsive to the treatment with an epothilone, determining the expression levels of multiple genes or proteins encoded by them in said mouse, and further identifying a marker protein or a gene coding for said protein by comparing the expression level of said gene or protein encoded by it in at least one xenograft tumor on mice or rats or tissue from these animals that is responsive to said epothilone treatment with the expression level of the same gene or protein in at least one xenograft tumor on mice or rats or tissue from these animals that is non-responsive to said epothilone treatment, alternatively wherein the expression level is compared to a reference standard in a non-malignant tissue, whereby the marker protein has an altered expression level in the responder compared to the non-responder, or compared to said reference standard expression level.

14. The method of claim 13, wherein the expression level of said multiple genes is obtained by the use of a microarray, preferably by the use of a Protein-, RNA- or cDNA-based microarray, or an SNP array.

15. Use of a nucleic acid coding for a marker protein or a fragment thereof in a method for determining potential responsiveness of a subject to treatment of a pathologic disorder with an epothilone, wherein the marker protein is selected from the group consisting of a cytochrome P (CYP) isoform, microsomal epoxide hydrolase, cytoplasmic epoxide hydrolase, p53, pro-apoptotic Fas-interacting partner (DAXX), neuregulin 1 (NRG1), hepatocyte growth factor (HGF), dystrophin, UGT1A1, UGT1A3, UGT1A4, UGT1A8, UGT1A10, AKR1C2, VEGF, GLUT1, aldolaseA, heme oxygenase, NIP3, PGK1, transferrin, and HIF-prolyl hydroxylase, or a nucleic acid having a complementary sequence thereto.

16. The use of claim 15, wherein the method for determining potential responsiveness of a subject to treatment of a pathologic disorder with an epothilone is carried out as defined in any one of claims 1 to 10.

17. Use of a nucleic acid hybridizing under stringent conditions to the gene coding for a marker protein selected from the group consisting of a cytochrome P (CYP) isoform, microsomal epoxide hydrolase, cytoplasmic epoxide hydrolase, p53, pro-apoptotic Fas-interacting partner (DAXX), neuregulin 1 (NRG1), hepatocyte growth factor (HGF), dystrophin, UGT1A1, UGT1A3, UGT1A4, UGT1A8, UGT1A10, AKR1C2, VEGF, GLUT1, aldolaseA, heme oxygenase, NIP3, PGK1, transferrin, and HIF-prolyl hydroxylase in a method as defined in any one of claims 1 to 10, or a nucleic acid having a complementary or homologous sequence thereto.

18. The use of claim 17, wherein the nucleic acid has a sequence that is complementary to the coding strand sequence of the gene coding for said marker protein.

19. The use of claim 17 or claim 18, wherein the nucleic acid is immobilized on a microarray, preferably wherein the microarray is a Protein-, RNA- or cDNA-based microarray, or an SNP array.

20. Use of an antibody against a marker protein selected from the group consisting of a cytochrome P (CYP) isoform, microsomal epoxide hydrolase, cytoplasmic epoxide hydrolase, p53, pro-apoptotic Fas-interacting partner (DAXX), neuregulin 1 (NRG1), hepatocyte growth factor (HGF), dystrophin, UGT1A1, UGT1A3, UGT1A4, UGT1A8, UGT1A10, AKR1C2, VEGF, GLUT1, aldolaseA, heme oxygenase, NIP3, PGK1, transferrin, and HIF-prolyl hydroxylase, in a method for determining potential responsiveness of a subject to treatment of a pathologic disorder with an epothilone.

21. The use of claim 20, wherein the method for determining potential responsiveness of a subject to treatment of a pathologic disorder with an epothilone is carried out as defined in claim 11 or claim 12.

22. The use of claim 20 or claim 21, wherein the antibody is selected from the group consisting of a monoclonal antibody, a polyclonal antibody, a partially humanized antibody, a fully humanized antibody, or an antibody-derived binding moiety, optionally wherein the antibody or an antibody-derived binding moiety further comprises a detectable label, preferably wherein the label is selected from a radioactive label and a fluorescent label.

23. Kit for determining the potential responsiveness of a subject to treatment of a pathologic disorder with an epothilone, comprising at least one nucleic acid as defined in any one of claims 15 to 18.

24. The kit of claim 23, comprising at least 2, preferably at least 5, more preferably at least 10, and most preferably at least 15 nucleic acids, wherein each nucleic acid codes for a single protein.

25. Kit for determining the potential responsiveness of a subject to treatment of a pathologic disorder with an epothilone, comprising at least one antibody as defined in any one of claims 20 to 22.

26. The kit of claim 25, comprising at least 2, preferably at least 5, more preferably at least 10, and most preferably at least 15 nucleic acids, wherein each nucleic acid codes for a single protein.

27. The kit of any one of claims 23 to 26, wherein the kit further comprises instructions for using the kit in a diagnostic method as defined in any one of claims 1 to 12.

28. A method of treating a subject suffering from a pathological disorder, comprising:
a) analyzing the gene expression profile of said subject to determine whether the subject will respond to treatment with an epothilone by a method as defined in any one of claims 1 to 12, and
b) treating the subject with an epothilone if the analysis indicates that the subject will respond to the treatment with said epothilone.

29. The method, use or kit according to any one of the preceding claims, wherein the pathologic disorder is selected from the group consisting of a malignant disorders, tumor diseases, inflammatory diseases, neurodegenerative diseases, angiogenesis-associated diseases, multiple sclerosis, Alzheimer's disease, osteoporosis, bone diseases, or rheumatoid arthritis.

30. The method, use or kit of claim 29, wherein said malignant disorder is selected from the group consisting of carcinoma, melanoma, lymphoma, sarcoma, and leukemia, preferably wherein said malignant disorder is selected from lung cancer, ovarian cancer and breast cancer, and most preferably wherein the malignant disorder is non-small cell lung cancer (NSCLC).

31. Use of an epothilone for the manufacture of a pharmaceutical for the treatment of a subject suffering from a pathological disorder selected from malignant disorders, tumor diseases, inflammatory diseases, neurodegenerative diseases, angiogenesis-associated diseases, multiple sclerosis, Alzheimer's disease, osteoporosis, bone diseases, or rheumatoid arthritis, wherein the subject to be treated has been determined to be responsive to a treatment with said epothilone.

32. The use of claim 31, wherein the responsiveness of said subject has been determined by a method of any one of claims 1 to 12.

33. Use of an epothilone for the manufacture of a pharmaceutical for the treatment of a tumor **characterized by** an altered expression of at least one gene selected from the group consisting of a cytochrome P (CYP) isoform, microsomal epoxide hydrolase, cytoplasmic epoxide hydrolase, p53, pro-apoptotic Fas-interacting partner (DAXX), neuregulin 1 (NRG1), hepatocyte growth factor (HGF), dystrophin, UGT1A1, UGT1A3, UGT1A4, UGT1A8, UGT1A10, AKR1C2, VEGF, GLUT1, aldolaseA, heme oxygenase, NIP3, PGK1, transferrin, and HIF-prolyl hydroxylase.

34. The use of claim 33, wherein the tumor is **characterized by** a mutated (loss of function) p53 gene, or wherein the tumor is **characterized by** an increased gene expression of DAXX and/or dystrophin.

35. The use of any one of claims 33 to 34, wherein the tumor is **characterized by** a decreased or at least non-increased expression of a cytochrome P (CYP) isoform, microsomal epoxide hydrolase, cytoplasmic epoxide hydrolase, neuregulin 1 (NRG1), hepatocyte growth factor (HGF), UGT1A1, UGT1A3, UGT1A4, UGT1A8, UGT1A10, AKR1C2, VEGF, GLUT1, aldolaseA, heme oxygenase, NIP3, PGK1, transferrin, or HIF-prolyl hydroxylase compared to the expression level in an epothilone non-responder or in a non-tumor tissue.

36. The use of any one of claims 31 to 35, wherein the pharmaceutical further comprises at least one other therapeutic agent known to be effective in treating said pathological disorder.

37. The use of claim 36, wherein the at least one other therapeutic agent is known to modulate the activity level of a pathway involved in said disorder, preferably wherein the at least one other therapeutic agent is selected from an antineoplastic agent, an anti-inflammatory agent, a neuroprotecting agent, an agent effective in the treatment of angiogenesis-associated diseases, multiple sclerosis, Alzheimer's disease, osteoporosis, bone diseases, or rheumatoid arthritis.

38. The use of claim 37, wherein said at least one other therapeutic agent triggers enhanced cell death (apoptosis) and necrosis.

39. The use of claim 37, wherein the antineoplastic agent is selected from the group consisting of etoposide, cisplatin, carboplatin, gemcitabine, capecitabine, fluorouracil, taxol, docetaxel, navelbine, cetuximab, erlotinib, vinorelbine, and mitomycin, drugs targeting kinases such as EGFR-inhibitors lapatinib, or growth factor antibodies herceptin, cetuximab, VEGFR-kinases such as sorafenib, sutent, IGFR-Kinases, mTOR inhibitors, HDAC-inhibitors such as SAHA, proteasome inhibitors such as velcade, steroid receptor antagonists such as antiestrogens, aromatase inhibitors, antiandrogens, DNA-methyltransferase inhibitors such as azacytidine, and the like.

40. Use of an epothilone and at least one other therapeutic agent for the manufacture of a pharmaceutical composition for the treatment of a subject suffering from a pathological disorder selected from malignant disorders, tumor diseases, inflammatory diseases, neurodegenerative diseases, angiogenesis-associated diseases, multiple sclerosis, Alzheimer's disease, osteoporosis, bone diseases, or rheumatoid arthritis, wherein the subject to be treated is determined to be non-responsive to a treatment with said epothilone in accordance with a method as defined in any one of claims 1 to 12, and further wherein the at least one other therapeutic agent is capable of modulating the activity level of a pathway that is indicative of a non-responsiveness of said subject to the treatment with an epothilone.

41. The use of claim 40, wherein the at least one other therapeutic agent is capable of reducing the expression level of at least one gene coding for a protein selected from the group consisting of a CYP isoform, microsomal epoxide hydrolase, cytoplasmic epoxide hydrolase, NRG1, HGF, UGT1A1, UGT1A3, UGT1A4, UGT1A8, UGT1A10, AKR1C2, VEGF, GLUT1, aldolaseA, heme oxygenase, NIP3, PGK1, transferrin, and HIF-prolyl hydroxylase.

42. The method, use or kit according to any one of the preceding claims, wherein said epothilone is selected from the group consisting of epothilone A, epothilone B, epothilone C, 13-alkyl-epothilone C derivatives, epothilone D, trans-epothilone D, epothilone E, epothilone F, an effector conjugate of epothilone, ZK-EPO (sagopilone), ixabepilone (BMS-247550), BMS-310705, EPO-906, Patupilone, Kos-862, Kos-1584, Kos-1803, ABJ 879, or a pharmaceutically acceptable salt thereof.
